(19) Europäisches Patentamt
European Patent Office
Office européen des brevets

(11) EP 0 564 477 B1

(12) **FASCICULE DE BREVET EUROPEEN**

(45) Date de publication et mention de la délivrance du brevet:
**04.11.1998 Bulletin 1998/45**

(21) Numéro de dépôt: **92900187.3**

(22) Date de dépôt: **20.11.1991**

(51) Int Cl.$^{6}$: **C07K 4/02**, C07K 14/15, A61K 39/21, G01N 33/569

(86) Numéro de dépôt international:
**PCT/FR91/00917**

(87) Numéro de publication internationale:
**WO 92/09632 (11.06.1992 Gazette 1992/13)**

(54) **FRAGMENTS DE LA PROTEINE ENV DU VIRUS DE L'IMMUNODEFICIENCE FELINE, ANTICORPS ET LEURS APPLICATIONS DANS LE DIAGNOSTIC**

PEPTIDFRAGMENTE DES ENV-PROTEINS VOM IMMUNSCHWÄCHE-VIRUS DER KATZE UND IHRE VERWENDUNG IN DER DIAGNOSTIK

FRAGMENTS OF THE ENV PROTEIN OF THE FELINE IMMUNODEFICIENCY VIRUS, ANTIBODIES AND THEIR APPLICATIONS IN DIAGNOSIS

(84) Etats contractants désignés:
**AT BE CH DE GB LI LU NL**

(30) Priorité: **21.11.1990 FR 9014519**

(43) Date de publication de la demande:
**13.10.1993 Bulletin 1993/41**

(73) Titulaire: **CENTRE NATIONAL DE LA RECHERCHE SCIENTIFIQUE (CNRS)**
**75794 Paris Cédex 16 (FR)**

(72) Inventeurs:
- **AVRAMEAS, Alexandre**
  **F-94400 Vitry-sur-Seine (FR)**
- **GUILLET, Jean, Gérard**
  **F-94450 Limeil-Brevannes (FR)**
- **MORAILLON, Anne**
  **F-94210 La Varenne-Saint-Hilaire (FR)**
- **STROSBERG, Arthur, Donny**
  **F-75015 Paris (FR)**

(74) Mandataire: **Ores, Irène et al**
**CABINET ORES**
**6, Avenue de Messine**
**75008 Paris (FR)**

(56) Documents cités:
- **PROC. NATL. ACAD. SCI. U.S.A., Vol. 86, October 1989, Washington, US, pages 8088-8092, R.A. OLMSTED et al., "Nucleotide Sequence Analysis of Feline Immunodeficiency Virus: Genome Organisation and Relationship to Other Lentiviruses".**
- **PROC. NATL. ACAD. SCI. U.S.A., Vol. 86, August 1989, Washington, US, pages 5743-5747, R.L. TALBOTT et al., "Nucleotide Sequence and Genomic Organisation of Feline Immunodeficiency Virus".**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 564 477 B1

## Description

La présente invention est relative à des fragments peptidiques issus de la protéine d'enveloppe du VIF (virus de l'immunodéficience féline) et à leur application dans le diagnostic de l'immunodéficience féline ; la présente invention est également relative à des anticorps anti-fragments peptidiques ainsi qu'à leurs applications au diagnostic et au traitement de l'immunodéficience féline.

L'immunodéficience féline est due à un lentivirus, le virus de l'immunodéficience féline (VIF), qui présente une structure génétique similaire à celle des lentivirus des primates (VIH et VIS).

L'étude de l'immunodéficience féline, notamment dans les populations de chats domestiques, peut permettre, d'une part un diagnostic rapide de cette maladie chez ces animaux et d'autre part, un traitement vis-à-vis de cette maladie ainsi qu'un modèle d'évaluation d'immunoconjugués vis-à-vis de cellules infectées par le VIF.

En effet, à l'heure actuelle, l'immunodéficience féline est essentiellement diagnostiquée lors de l'apparition des signes cliniques (lymphadénopathie généralisée et augmentation de la survenue d'infections opportunistes), alors qu'un diagnostic très précoce aurait des avantages importants tant dans le traitement que dans la prévention de cette maladie chez les chats domestiques, qui représentent une population très importante.

R.L. TALBOTT et al. (Natl. Acad. Sci. USA, 1989, 86, 5743-5747) ont plus particulièrement décrit la séquence nucléotidique de la souche *Petaluma,* et précisent que le provirus intégré a une longueur totale de 9 472 paires de bases et possède la capacité de codage pour plusieurs protéines ; en particulier il est précisé que la région *gag* code pour les antigènes du coeur du virus, la région *pol* code pour une protéase, une transcriptase réverse et une endonucléase et la région *env* code pour les glycoprotéines majeures et mineures de l'enveloppe virale.

R.A. OLMSTED et al. (Proc. Natl. Acad. Sci. USA, vol. 86, p. 8088-8092, octobre 1989) ont, par ailleurs, décrit la séquence d'un clone d'ADN proviral infectieux (VIF-14), du VIF.

T. MIYAZAWA et al. (Arch. Virol., 1989, 108, 59-68) ont décrit deux souches de VIF, d'origine japonaise, dénommées VIF TM 1 et VIF TM 2 ; les protéines structurales de TM 1 et de TM 2 ont été comparées en analyse par immunoblotting avec celles de la souche *Petaluma* ; les souches TM 1 et TM 2 présentent un certain nombre de protéines de structure communes avec la souche *Petaluma* (bandes 48, 44, 40, 28 et 17 kDa) et deux protéines de structure distinctes (bandes à 130 kDA et à 13 kDa).

T. MIYAZAWA et al. (Arch. Virol., 1989, 108, 131-135) ont mis au point une lignée cellulaire permettant la réplication du VIF ; il s'agit de cellules lymphoblastiques T félines, dépendantes de l'interleukine 2 et dénommées cellules MYA-1.

La Demande Internationale PCT WO 90/13573 décrit de manière générale des polypeptides purifiés ayant un épitope d'un polypeptide antigénique de VIF. Ces polypeptides ont au moins 75 % d'homologie avec des fragments d'au moins 20 aminoacides obtenus à partir d'un polypeptide *gag* ou *env de* VIF (p10, p26, gp40, gp100 et gp130).

Bien qu'un certain nombre d'études aient été réalisées, notamment en ce qui concerne la souche *Petaluma* du VIF, le diagnostic précoce de l'immunodéficience féline est actuellement fort coûteux ; de plus, les kits existants sont très peu sensibles et leur manipulation comporte des risques biologiques pour l'utilisateur, par exemple le kit "PETCHECK" (AGRITECH SYSTEMS INC.), dans lequel les plaques de microtitration sont recouvertes de FIV inactivé chimiquement.

La présente invention s'est en conséquence donné pour but de pourvoir à des fragments peptidiques, issus d'une protéine env du VIF qui répondent mieux aux besoins de la pratique, notamment en permettant la mise au point de tests diagnostics hautement spécifiques ne comportant pas de risques pour l'utilisateur, permettant un diagnostic préclinique de l'immunodéficience féline et peu coûteux, ainsi que la préparation d'anticorps à visée thérapeutique et/ou préventive et/ou diagnostique, également hautement spécifiques d'un épitope immunodominant de l'immunodéficience féline due au VIF.

La présente invention a pour objet un fragment peptidique comprenant une portion d'une protéine env du virus de l'immunodéficience féline (VIF) comprise entre l'aminoacide 66 et l'aminoacide C terminal de la protéine *env,* caractérisé en ce qu'il est sélectionné dans le groupe constitué par :

- un fragment peptidique qui comprend 19 amino-acides, contient au moins un épitope immunogène majeur de ladite protéine *env* du VIF et présente la formule I suivante :

```
Asn-Gln-Thr-Cys-Met-Trp-Asn-Thr-Ser-Gln-Ile-Gln-Asp-Pro-
Glu-Ile-Pro-Lys-Cys.                                  (I),
```

  qui correspond aux positions 336-355 de la protéine *env* de la souche *Petaluma,*
- un fragment peptidique qui comprend 21 aminoacides, contient au moins un épitope immunogène majeur de ladite protéine env du VIF et présente la formule II suivante :

Leu-Val-Val-Pro-Glu-Glu-Val-Met-Glu-Tyr-Lys-Pro-Arg-Arg-Lys-Arg-Ala-Ala-Ile-His-Val. (II),

qui correspond aux positions 596-616 de la protéine *env* de la souche *Petaluma* et dans laquelle X représente Leu ou Val,

- un fragment peptidique qui comprend 25 aminoacides, contient au moins un épitope immunogène majeur de ladite protéine env du VIF et présente la formule III suivante :

Val-Ile-Ala-Met-Pro-Glu-Val-Glu-Gly-Glu-Glu-Ile-Gln-Pro-Gln-Met-Glu-Leu-Arg-Arg-Asn-Gly-Arg-Gln-Cys (III),

qui correspond aux positions 824-848 de la protéine *env* de la souche *Petaluma,*

- un fragment peptidique qui comprend 26 amino-acides et présente la formule IV suivante :

Leu-Arg-Asn-Glu-Ile-Gln-Glu-Val-Lys-Leu-Glu-Glu-Gly-Asn-Ala-Gly-Lys-Phe-Arg-Arg-Ala-Arg-Phe-Leu-Arg-Tyr (IV),

qui correspond aux positions 66-91 de la protéine *env* de la souche *Petaluma,* et

- un fragment peptidique qui comprend 28 amino-acides et présente la formule V suivante :

Leu-Gln-Gly-Lys-Ile-Asn-Ile-Ser-Leu-Cys-Leu-Thr-Gly-Gly-Lys-Met-Leu-Tyr-Asn-Lys-Val-Thr-Lys-Gln-Leu-Ser-Tyr-Cys (V),

qui correspond aux positions 292-320 de la protéine *env* de la souche *Petaluma,*

lesquels fragments sont reconnus au moins par les anticorps produits lors d'une infection et/ou lors d'une inoculation par l'une quelconque des souches de VIF.

Le fragment peptidique de formule I est dénommé ci-après peptide 99 (P99), le fragment peptidique de formule II est dénommé ci-après peptide 100 (P100), le fragment peptidique de formule III est dénommé ci-après peptide 102 (P102), le fragment peptidique de formule IV est dénommé ci-après peptide 101 (P101), et le fragment peptidique de formule V est dénommé ci-après peptide 103.

On entend par protéine *env,* l'ensemble de ladite protéine, également dénommée protéine de surface (SU), qui après clivage peut fournir les deux fragments, dénommés gp120 et gp41 (partie dite transmembranaire de la protéine (TM)).

On entend, au sens de la présente invention, par fragment peptidique aussi bien les séquences d'amino-acides telles que définies ci-dessus, que ces mêmes séquences modifiées (sites potentiels de glycosylation, acylation notamment).

Ces différents fragments peptidiques peuvent notamment être obtenus par synthèse, en particulier par la méthode de Merrifield (Science, 1965, 150, 178-184). Lesdits fragments peptidiques peuvent également être préparés par génie génétique (expression du gène approprié chez les procaryotes ou les eucaryotes).

La présente invention a également pour objet des anticorps anti-fragment peptidique, caractérisés en ce qu'ils sont obtenus par immunisation d'un animal approprié avec au moins un fragment peptidique conforme à l'invention, lesquels anticorps présentent une affinité pour ledit peptide, et sont aptes à reconnaître la protéine *env* du VIF, le VIF et/ou les cellules exprimant le VIF.

Les anticorps monoclonaux anti-fragment peptidique sont avantageusement obtenus, d'une manière connue en elle-même, par fusion de cellules spléniques de souris immunisées par un antigène constitué par un fragment peptidique tel que défini ci-dessus, avec des cellules myélomateuses appropriées.

Selon un autre mode de réalisation, les fragments peptidiques sont avantageusement ceux qui comprennent un épitope immunogène majeur et notamment les fragments P99, P100 ou P102 tels que décrits ci-dessus.

La présente invention a également pour objet un réactif immunologique utilisable pour la détection, le diagnostic et le suivi de l'immunodéficience féline, caractérisé en ce qu'il est choisi dans le groupe constitué par les fragments peptidiques et les anticorps anti-peptide conformes à l'invention, éventuellement associés à une autre substance, notamment une enzyme.

Selon un mode de mise en oeuvre avantageux dudit réactif, il est constitué par un mélange des peptides conformes à l'invention.

La présente invention a également pour objet un procédé de détection et/ou de diagnostic de l'immunodéficience féline, caractérisé en ce qu'il consiste à détecter les anticorps éventuellement présents dans un fluide biologique tel que le sang, en mettant en présence ledit fluide avec au moins un fragment peptidique conforme à l'invention, auquel se lient les anticorps anti-VIF, si de tels anticorps sont présents dans l'échantillon biologique à contrôler, la lecture du résultat étant révélé par un moyen approprié, notamment RIA, EIA ou cytométrie en flux.

Le procédé conforme à l'invention, utilisant les fragments peptidiques ci-dessus définis, notamment utilisés en *pool,* permet d'une part d'obtenir une sensibilité nettement améliorée par rapport aux procédés de l'Art antérieur, dans la mesure où l'on peut obtenir un résultat facilement interprétable avec des sérums dilués jusqu'au 1/200ème et d'autre part, d'effectuer les dosages à partir de très petites quantités d'échantillons. L'utilisation d'un *pool* de peptides présente en outre l'avantage d'éviter les résultats faussement négatifs ou faussement positifs, rencontrés dans les procédés de l'Art antérieur ; de plus de tels peptides sont de manipulation moins dangereuse que les réactifs proposés dans l'Art antérieur.

La présente invention a également pour objet un kit ou trousse de diagnostic pour la détection, le diagnostic ou le suivi de l'immunodéficience féline, caractérisé en ce qu'il comprend :

- un support solide approprié revêtu d'au moins un fragment peptidique conforme à l'invention,
- des quantités ou doses appropriées de conjugué choisi dans le groupe qui comprend les conjugués enzyme appropriée-anticorps anti-Ig félins appropriés et les conjugués enzyme appropriée-anticorps anti-peptide conforme à l'invention,
- des quantités ou doses appropriées d'une substance de révélation convenable.

On peut citer notamment comme substance de révélation appropriée, notamment l'ABTS.

On peut citer comme support approprié, notamment des plaques de microtitration NUNC-IMMUNO PLATE MAXISORP®, NUNC-IMMUNO IP® ou DYNATECH IMMULON 2® ; toutefois d'autres supports tels que des billes, des sticks, des filtres, des bandelettes pourraient également être utilisés.

On peut citer comme enzyme appropriée, notamment la peroxydase, la phosphatase alcaline ou toute autre enzyme utilisable comme marqueur dans un test ELISA.

Dans le cas d'une méthode directe, par exemple, les anticorps anti-VIF du chat se fixent au peptide conforme à l'invention, préalablement fixé au support solide et des conjugués enzyme appropriée-anticorps-anti-Ig félins appropriés par exemple, et ce de manière non limitative, de la peroxydase liée à l'anticorps de chèvre anti-IgG (H+L) de chat (KIRKEGAARD & PERRY LABORATORIES) sont introduits, se lient avec les Ig du chat et sont ensuite révélés de manière appropriée.

Dans le cas d'une méthode indirecte, par exemple, des conjugués enzyme appropriée-anticorps anti-peptide conforme à l'invention entrent en compétition avec les anticorps du chat infecté, pour se lier au support solide revêtu d'un peptide conforme à l'invention.

La présente invention a, de plus, pour objet un procédé d'identification de souche de VIF, caractérisé en ce que l'on met en contact un échantillon à analyser, notamment un fluide biologique avec un panel de fragments peptidiques conformes à l'invention, et en ce que l'on détermine par tout moyen approprié le/les fragments peptidiques qui se sont éventuellement liés aux anticorps produits par la souche à détecter.

La présente invention a également pour objet des agents à visées thérapeutiques et/ou préventives, caractérisés en ce qu'ils sont constitués par, ou comprennent en tant que constituant actif, un peptide conforme à l'invention, seul ou conjugué ou associé à d'autres substances.

La présente invention a également pour objet des agents à visée thérapeutique et/ou préventive, caractérisés en ce qu'ils sont constitués par, ou comprennent en tant que constituants actifs, des anticorps anti-peptide conformes à l'invention, seuls ou conjugués avec d'autres substances.

Selon un mode de réalisation avantageux desdits agents, ils sont associés à une enzyme oxydative.

Selon une disposition avantageuse de ce mode de réalisation, l'enzyme oxydative est choisie dans le groupe qui comprend les enzymes oxydatives générant de l'oxygène actif et les enzymes oxydatives dont le substrat est de l'oxygène actif et un halogénure.

Selon une modalité avantageuse de cette disposition, les enzymes oxydatives générant de l'oxygène actif sont choisies dans le groupe qui comprend la glucose oxydase (GO), la NADPH oxydase, la xanthine oxydase, notamment la xanthine oxydase du lait, la cholestérol oxydase, la choline oxydase, l'acide lactique oxydase, la pyruvate oxydase.

Selon une autre modalité avantageuse de cette disposition, les enzymes oxydatives dont le substrat est de l'oxygène actif et un halogénure sont choisies dans le groupe qui comprend la lactoperoxydase (LPO), la myéloperoxydase, notamment la myéloperoxydase des neutrophiles, la peroxydase des éosinophiles, et la peroxydase salivaire.

Le terme conjugué signifie que l'enzyme et le ligand sont connectés par des liaisons covalentes. Ceci est accompli par des moyens connus en soi et généralement appliqués aux anticorps marqués avec des enzymes pour les tests ELISA, typiquement en utilisant des réactifs de liaison bifonctionnelle tels que le SDDP, et le 2-iminothiolane.

De tels immunoconjugués peuvent également être obtenus en couplant, par génie génétique, les gènes $V_H$ et $V_L$ des anticorps avec les gènes de l'enzyme appropriée.

De tels immunoconjugués et notamment une utilisation en tandem (anticorps-enzymes oxydatives générant de l'oxygène actif, GO par exemple et anticorps-enzymes oxydatives dont le substrat est de l'oxygène actif et un halogénure, LPO par exemple) permet de détruire spécifiquement les cellules infectées par le VIF, tout en réduisant la destruction de cellules non ciblées, tant *in vitro* qu'*in vivo.*

Dans le cas du tandem anticorps-GO/anticorps-LPO, par exemple, le principe de base de l'utilisation de ces immunoconjugués est qu'en présence de glucose, la glucose oxydase produit l'$H_2O_2$ qui est le substrat de la lactoperoxydase. Cette enzyme catalyse ensuite l'oxydation du NaI (ajouté au milieu au même titre que le glucose) en radicaux libres. Les radicaux libres étant hautement toxiques pour les cellules, ils détruisent un grand nombre d'espèces moléculaires indispensables au fonctionnement et à la multiplication des cellules.

La présente invention a, de plus, pour objet une méthode d'évaluation de l'efficacité d'immunoconjugués, caractérisé en ce qu'elle comprend :

- des cellules de chat aptes à développer le VIF et/ou infectées ou inoculées par ledit virus, et
- des immunoconjugués comprenant des enzymes oxydatives couplées à des anticorps choisis dans le groupe qui comprend les anticorps anti-VIF et les anticorps anti-peptide conformes à l'invention.

Conformément à l'invention, les cellules aptes à développer le VIF et/ou infectées ou inoculées par ledit virus sont notamment des cellules mononucléaires de chats infectés, des cellules lymphoïdes primaires et des lignées lymphoblastiques T félines, notamment les lignées MYA-1, telles que décrites par T. MIYAZAWA et al..

Une telle méthode est notamment utilisable pour évaluer l'efficacité desdits immunoconjugués vis-à-vis notamment du virus de l'immunodéficience humaine (VIH).

La présente invention a également pour objet un procédé d'évaluation de l'efficacité d'immunoconjugués, caractérisé en ce que des immunoconjugués comprenant des enzymes oxydatives couplées à un anticorps approprié choisi dans le groupe qui comprend les anticorps anti-VIF et les anticorps anti-peptide conformes à l'invention sont mis en contact avec des cellules de chat aptes à développer le VIF et/ou infectées ou inoculées par ledit virus et en ce que la toxicité desdits immunoconjugués vis-à-vis desdites cellules, dites cellules cibles, est mesurée par tout moyen approprié.

Un tel procédé est notamment applicable à l'évaluation de l'efficacité desdits immunoconjugués vis-à-vis notamment du VIH.

Outre les dispositions qui précèdent, l'invention comprend encore d'autres dispositions qui ressortiront de la description qui va suivre, qui se réfère à des exemples de mise en oeuvre du procédé objet de la présente invention, avec référence aux figures annexées dans lesquelles :

- la figure 1 représente les séquences des peptides 99, 100, 101, 102 et 103, et leurs positions sur la protéine *env* de surface (SU), soit dans la portion gp120, soit dans la portion gp41 (partie dite transmembranaire de la protéine (TM)) ;
- la figure 2 représente le délai d'apparition des anticorps dans le sérum de chats inoculés avec deux souches de VIF (W.O. et M.E.); cette figure met en valeur la reconnaissance des peptides 102 et 100 par les sérums desdits chats ;
- les figures 3 à 7 représentent des courbes de titration d'anticorps anti-peptides.

Il doit être bien entendu, toutefois, que ces exemples et ces figures sont donnés uniquement à titre d'illustration de l'objet de l'invention, dont ils ne constituent en aucune manière une limitation.

**<u>EXEMPLE 1</u> : Test témoin : ELISA réalisé sur des sérums de chats non infectés.**

a. Protocole opératoire

50 µl d'une solution contenant 0,25 µg d'un peptide contenant un épitope immunodominant de la protéine env du VIF (peptides 99, 100 ou 102) ou d'un peptide dit témoin négatif, c'est-à-dire ne contenant pas d'épitope immunodominant de la protéine *env* du VIF (peptides 101 et 103), dans du carbonate de sodium 0,1 M, pH 9,6 sont adsorbés sur les puits d'une plaque de microtitration NUNC-IMMUNO IP® ou DYNATECH IMMULON 2® contenant 96 puits pendant une nuit à 4°C.

Les puits sont lavés 3 fois avec un tampon phosphate à pH 7,4 (PBS). Les sites d'adsorption résiduels sur les plaques de microtitration sont saturés par incubation avec 100 µl de PBS contenant 1 % de lait ou de gélatine et du Tween 20 (0,1 %) pendant 1 à 3 heures. 50 µl de sérum à tester dilué du 1/20ème au 1/200ème, de préférence au 1/50ème (pour la présence éventuelle d'anticorps anti-VIF) est incubé dans les puits pendant 2 heures à température ambiante ou une nuit à 4°C. Après 3 lavages avec un mélange de PBS, lait ou gélatine 1 % et Tween 20 0,1 %, on ajoute dans les puits un conjugué peroxydase-anticorps anti-IgG (H+L) de chat ou de lapin (0,5 µg/ml) et on incube 1 heure à température ambiante. Après lavage de la plaque de microtitration cinq fois avec du PBS, le conjugué peroxydase adsorbé est visualisé avec un substrat consistant en 2,2'-azino-bis(3-éthyl-benzthiazoline-6-acide sulfonique) (ABTS) 0,4 mM final dans un tampon acétate 0,6 % final, pH 4,7 et 1 µl d'$H_2O_2$ à 30 % (v/v) par 1 ml de milieu de réaction.

Lorsque l'enzyme du conjugué est de la glucose oxydase, la visualisation est réalisée directement avec un substrat consistant en 300 µl de glucose (18 %), de l'ABTS 0,4 mM final dans un tampon acétate 0,6 % final, pH 4,7 et 2 µg de peroxydase.

Le produit de réaction est mesuré à l'aide d'un colorimètre, à 405 nm.

Le test ELISA lui-même est mis en oeuvre comme spécifié dans l'article paru dans Immunoenzym. Techn., 1983, p. 307, au nom de HOEBEKE J. et al. (S. AVRAMEAS et al. Editeurs).

b. Résultats

TABLEAU I

| SERUMS | Peptide 99 | Peptide 100 | Peptide 101 | Peptide 102 | Peptide 103 |
|---|---|---|---|---|---|
| Chats non infectés par le VIF (témoins) | | | | | |
| CTD[1] 28652[2] | - | - | - | - | - |
| CTD 28629 | - | - | - | - | - |
| CTD 28632 | - | - | - | - | - |
| CTD 28623 | - | - | - | - | - |
| CTD 29409 | - | - | - | - | - |
| CTD 29498 | - | - | - | - | - |
| CTD 29920 | - | - | - | - | - |
| CTD 29925 | - | - | - | - | - |
| CTD 29939 | - | - | - | - | - |
| CTD 29941 | - | - | - | - | - |
| CTD 29944 | - | - | - | - | - |

[1] Chat diagnostiqué
[2] numéro de sérum

Il ressort de ce Tableau que les chats non infectés, ne produisent pas d'anticorps anti-VIF dans leur sérum ; il n'y a donc aucune liaison avec les différents fragments peptidiques.

**EXEMPLE 2 : Test ELISA réalisé chez des chats infectés par le VIF.**

a. Protocole opératoire

Le protocole opératoire est identique à celui de l'exemple 1.

b. Résultats

TABLEAU II

| SERUMS | Peptide 99 | Peptide 100 | Peptide 101 | Peptide 102 | Peptide 103 |
|---|---|---|---|---|---|
| Chats infectés naturellement par le VIF : | | | | | |
| CTD[1] 28261[2] | - | + | - | + | - |
| CTD 28518 | - | + | - | + | - |
| CTD 28526 | - | + | - | - | - |
| CTD 28554 | + | + | - | - | - |
| CTD 28565 | + | + | - | + | - |
| CTD 28646 | + | + | - | + | - |
| CTD 29376 | + | + | - | + | - |
| CTD 29290 | + | + | - | + | - |
| CTD 29494 | + | + | - | - | - |
| CTD 29536 | + | + | - | - | - |
| CTD 29459 | + | + | - | - | - |
| CTD 29463 | + | + | - | + | - |
| CTD 29412 | + | + | - | + | - |
| CTD 29590 | + | + | - | + | - |
| CTD 29542 | + | + | - | + | - |
| CTD 29576 | + | + | - | - | - |
| CTD 29560 | + | + | - | + | - |
| CTD 28888 | + | + | - | + | - |
| CTD 28889 | + | + | - | - | - |
| CTD 28905 | + | + | - | + | - |
| CTD 28912 | + | + | - | + | - |
| CTD 28797 | + | + | - | + | - |
| CTD 28827 | + | + | - | - | - |
| CTD 28845 | + | + | - | + | - |
| CTD 28921 | + | + | - | + | - |
| CTD 28933 | + | + | - | + | - |
| CTD 28943 | + | + | - | + | - |
| CTD 29029 | + | + | - | + | - |
| CTD 29864 | + | + | - | + | - |
| CTD 29940 | + | + | - | - | - |
| CTD 29942 | + | + | - | - | - |
| CTD 29945 | + | + | - | - | - |

[1] Chat diagnostiqué
[2] numéro de sérum

Il ressort de ce Tableau que les chats infectés naturellement, quelle que soit la souche et la phase de l'infection, produisent des anticorps anti-VIF dans leur sérum, lesquels anticorps reconnaissent les fragments peptidiques 99, 100 et 102 conformes à l'invention.

**<u>EXEMPLE 3</u> : Chats inoculés par la souche française de VIF, dénommée M.E**

a. Protocole opératoire

Le protocole opératoire est identique à celui de l'exemple 1.

b. Résultats

TABLEAU III

| SERUMS | Peptide 99 | Peptide 100 | Peptide 101 | Peptide 102 | Peptide 103 |
|---|---|---|---|---|---|
| Chats inoculés par la souche M. E : | | | | | |
| CTF[1] PLUa[2] | - | + | - | + | - |
| CTF PLUb | - | + | - | + | - |
| CTF PIA | - | + | - | + | - |
| CTF MAN | - | + | - | + | - |
| CTF BIAa | - | - | - | + | - |
| CTF BIAb | - | - | - | + | - |
| Chats avant inoculation: | | | | | |
| CTF PLUo | - | - | - | - | - |
| CTF BIAo | - | - | - | - | - |
| CTF MANo | - | - | - | - | - |
| CTF PIAo | - | - | - | - | - |

[1] Chats, souches françaises

[2] Initiales du chat : o, a, b correspondent à des durées d'infection différentes, pour un même chat.

**<u>EXEMPLE 4</u> : Chats inoculés par la souche française de VIF, dénommée W.O**

a. Protocole opératoire

Le protocole opératoire est identique à celui de l'exemple 1.

b. Résultats

TABLEAU IV

| SERUMS | Peptide 99 | Peptide 100 | Peptide 101 | Peptide 102 | Peptide 103 |
|---|---|---|---|---|---|
| Chats inoculés par la souche W. O : | | | | | |
| CTF[1] SOU[2] | - | + | - | + | - |
| CTF VRI | - | + | - | + | - |
| | - | + | - | + | - |
| Chats avant inoculation: | | | | | |
| CTF VRI | - | - | - | - | - |

[1] Chats, souches françaises

[2] Initiales du chat

**<u>EXEMPLE 5</u> : Chats inoculés par la souche *Petaluma.***

TABLEAU V

| SERUMS | Peptide 99 | Peptide 100 | Peptide 101 | Peptide 102 | Peptide 103 |
|---|---|---|---|---|---|
| Chats inoculés par la souche *Petaluma :* | | | | | |
| 2 mois après inoculation (7 G) : | - | + | - | + | - |
| 6 mois après inoculation (4 D) : | - | + | - | + | - |
| 6 mois après inoculation (10 J) : | - | + | - | + | - |
| 1 an après inoculation (140) : | - | + | - | + | - |

Les Tableaux III à V ci-dessus suggèrent les commentaires suivants :

- les anticorps produits par les chats inoculés par les différentes souches se lient aux peptides 100 et 102 conformes à l'invention,
- le peptide 99, conforme à l'invention, n'est pas reconnu par les anticorps produits contre des souches du VIF inoculés, alors qu'il est reconnu par des anticorps de sérums de chats infectés naturellement par le VIF.

P102 apparaît donc comme spécifique des chats inoculés ; P99 apparaît plutôt comme spécifique des chats infectés de manière naturelle alors que P100 pourrait être considéré comme un marqueur de l'infection au VIF.

**<u>EXEMPLE 6</u> : Préparation d'anticorps anti-peptides.**

a. préparation de l'antigène

Des conjugués P99, P100 et P102 avec soit la KLH, soit la BSA, sont préparés, en utilisant deux agents de couplage différents : le glutaraldéhyde (ZEGERS et al., J. Immunol. Methods, 1990, **130**, 195-200) ou le N-succinimidyl 4-maléimidobutyrate (FUJIWARA et al., J.Immunol. Methods, 1981, **45**, 195-203).

b. immunisation

Les conjugués KLH et BSA sont injectés à des lapins conformément aux protocoles d'immunisation établis (NIMAN et al., Proc. Natl. Acad. Sci. USA, 1983, **80**, 4949-4953).

**<u>EXEMPLE 7</u> : Titrations des premiers sérums (saignée après le premier rappel) de lapins immunisés avec les peptides P99 et P100 conjugués à la KLH ou à la BSA, les plaques de microtitration étant sensibilisées avec les peptides P99 et P100 libres.**

a. Protocole opératoire

Le protocole opératoire est identique à celui de l'exemple 1, à l'exception du fait que le conjugué est, en ce cas, un conjugué peroxydase-anticorps de chèvre anti-Ig de lapin.

b. Résultats :

TABLEAU VI

| DILUTIONS | P99-KLH[6]a | P99-KLH[5]b | P100-KLH[4] | P99-BSA[3] | TEMOIN P99[2] | TEMOIN P100[1] |
|---|---|---|---|---|---|---|
| 1/10 | 0,597 | 0,309 | 0,817 | 0,423 | 0,479 | 0,069 |
| 1/20 | 0,595 | 0,367 | 0,926 | 0,469 | 0,482 | 0,044 |
| 1/40 | 0,671 | 0,507 | 0,891 | 0,438 | 0,299 | 0,028 |
| 1/80 | 0,703 | 0,561 | 0,897 | 0,338 | 0,286 | 0,031 |
| 1/160 | 0,685 | 0,542 | 0,711 | 0,255 | 0,202 | 0,025 |
| 1/320 | 0,540 | 0,331 | 0,520 | 0,121 | 0,137 | 0,016 |
| 1/640 | 0,248 | 0,176 | 0,364 | 0,032 | 0,099 | 0,005 |
| 1/1280 | 0,091 | 0,085 | 0,266 | 0,023 | 0,065 | 0,003 |
| 1/2560 | 0,086 | 0,060 | 0,138 | 0,006 | 0,040 | 0,012 |
| 1/5120 | 0,044 | 0,000 | 0,097 | 0,000 | 0,035 | 0,011 |
| 1/10240 | 0,020 | 0,000 | 0,079 | 0,000 | 0,036 | 0,010 |
| 1/20480 | 0,004 | 0,000 | 0,041 | 0,000 | - | - |
| 1/40960 | 0,000 | 0,000 | 0,027 | 0,000 | - | - |
| Sensibilisation des plaques ELISA avec : | P99 libre | P99 libre | P100 libre | P99 libre | P100 libre | P99 libre |

[1] Test ELISA sur un lapin immunisé par le peptide 100 et sensibilisation de la plaque par le peptide 99.
[2] Test ELISA sur un lapin immunisé par le peptide 99 et sensibilisation de la plaque par le peptide 100.
[3] Lapin immunisé avec le peptide 99 couplé par le N-succinimidyl 4-maléimidobutyrate (GMBS) à la protéine porteuse BSA.
[4] Lapin immunisé avec le peptide 100 couplé par le glutaraldéhyde à la protéine porteuse KLH.
[5] Deuxième lapin immunisé avec le peptide 99 couplé par le glutaraldéhyde à la protéine porteuse KLH.
[6] Premier lapin immunisé avec le peptide 99 couplé par le glutaraldéhyde à la protéine porteuse KLH.

La figure 3 représente les résultats obtenus au Tableau VI ci-dessus, à savoir les titrations des premiers sérums de lapins immunisés avec les peptides P99 et P100 conjugués à la KLH ou à la BSA, les plaques de microtitration étant sensibilisées avec les peptides P99 et P100 libres.

Cette figure comprend en ordonnée les densités optiques à 405 nm et en abscisse les dilutions des sérums (échelle log). Les courbes (1) et (2) correspondent aux témoins P99 et P100, la courbe (3) fournit les titrations d'un sérum de lapin immunisé avec le conjugué P99-BSA, la courbe (4) fournit les titrations d'un sérum de lapin immunisé avec le conjugué P100-KLH et les courbes (5) et (6) fournissent les titrations d'un sérum de lapin immunisé avec le conjugué P99-KLH.

**<u>EXEMPLE 8</u> : Titrations des premiers sérums (saignée après le premier rappel) de lapin immunisés avec les peptides P99 et P100 conjugués à la KLH ou à la BSA, les plaques de microtitration étant sensibilisées avec les peptides P99 et P100 conjugués.**

TABLEAU VII

| DILUTIONS | P99-BSA[3] | P99-KLH a[6] | P99 KLH b[5] | P100-KLH[4] | TEMOIN P99[2] | TEMOIN P100[1] |
|---|---|---|---|---|---|---|
| 1/10 | 0,608 | 0,804 | 0,483 | 0,808 | 0,147 | 0,210 |
| 1/20 | 0,598 | 0,729 | 0,624 | 0,726 | 0,062 | 0,199 |
| 1/40 | 0,608 | 0,783 | 0,515 | 0,760 | 0,050 | 0,125 |
| 1/80 | 0,581 | 0,832 | 0,754 | 0,702 | 0,056 | 0,125 |
| 1/160 | 0,599 | 0,802 | 0,694 | 0,685 | 0,057 | 0,073 |
| 1/320 | 0,421 | 0,813 | 0,635 | 0,517 | 0,032 | 0,051 |

[1], [2], [3], [4], [5] et [6] ont la même signification qu'au Tableau VI ci-dessus.

TABLEAU VII (suite)

| DILUTIONS | P99-BSA[3] | P99-KLH a[6] | P99 KLH b[5] | P100-KLH[4] | TEMOIN P99[2] | TEMOIN P100[1] |
|---|---|---|---|---|---|---|
| 1/640 | 0,242 | 0,632 | 0,318 | 0,443 | 0,028 | 0,027 |
| 1/1280 | 0,208 | 0,436 | 0,315 | 0,297 | 0,015 | 0,030 |
| 1/2560 | 0,178 | 0,361 | 0,290 | 0,175 | 0,001 | 0,026 |
| 1/5120 | 0,101 | 0,197 | 0,214 | 0,125 | - | 0,013 |
| 1/10240 | 0,095 | 0,144 | 0,152 | 0,123 | - | 0,005 |
| 1/20480 | 0,045 | 0,048 | 0,151 | 0,049 | - | - |
| 1/40960 | 0,026 | 0,034 | 0,043 | 0,037 | - | - |
| 1/81920 | 0,023 | 0,020 | 0,041 | 0,027 | - | - |
| 1/163840 | 0,024 | 0,018 | 0,047 | 0,022 | - | - |
| 1/327680 | 0,026 | 0,025 | 0,031 | 0,021 | - | - |
| Sensibilisation des plaques avec : | P99-KLH a[7] | P99-BSA[8] | P99-BSA[8] | P100-BSA[9] | P100-BSA[9] | P99-BSA[8] |

[1], [2], [3], [4], [5] et [6] ont la même signification qu'au Tableau VI ci-dessus.

[7] Peptide 99 couplé par le glutaraldéhyde à la protéine porteuse KLH.

[8] Peptide 99 couplé par le N-succinimidyl 4-maléimidobutyrate (GMBS) à la protéine porteuse BSA.

[9] Peptide 100 couplé par le glutaraldéhyde à la protéine porteuse BSA.

La figure 4 représente les résultats obtenus au Tableau VII ci-dessus, à savoir les titrations des premiers sérums de lapins immunisés avec les peptides P99 et P100 conjugués à la KLH ou à la BSA, les plaques de microtitration étant sensibilisées avec les peptides P99 et P100 conjugués.

Cette figure comprend en ordonnée les densités optiques à 405 nm et en abscisse les dilutions des sérums (échelle log). Les courbes (1) et (2) correspondent aux témoins P99 et P100, la courbe (3) fournit les titrations d'un sérum de lapin immunisé avec le conjugué P99-BSA, la courbe (4) fournit les titrations d'un sérum de lapin immunisé avec le conjugué P100-KLH et les courbes (5) et (6) fournissent les titrations d'un sérum de lapin immunisé avec le conjugué P99-KLH.

**<u>EXEMPLE 9</u> : Titrations des deuxièmes sérums (saignée après le deuxième rappel des mêmes lapins que ceux des Exemples 6 et 7) de lapins immunisés avec les peptides P99 et P100 conjugués à la KLH ou à la BSA, sensibilisés avec les peptides P99 et P100 libres.**

TABLEAU VIII

| DILUTIONS | P99-KLH a[6] | P99-KLH b[5] | P100 KLH[4] | P99-BSA[3] | TEMOIN P99[2] | TEMOIN P100[1] |
|---|---|---|---|---|---|---|
| 1/10 | 0,815 | 0,700 | 0,817 | 0,423 | 0,127 | 0,240 |
| 1/20 | 0,812 | 0,725 | 0,978 | 0,666 | 0,110 | 0,133 |
| 1/40 | 0,820 | 0,732 | 0,952 | 0,626 | 0,065 | 0,092 |
| 1/80 | 0,775 | 0,708 | 0,960 | 0,593 | 0,056 | 0,063 |
| 1/160 | 0,752 | 0,702 | 0,879 | 0,584 | 0,065 | 0,049 |
| 1/320 | 0,750 | 0,689 | 0,801 | 0,573 | 0,045 | 0,035 |
| 1/640 | 0,535 | 0,565 | 0,739 | 0,498 | 0,045 | 0,031 |
| 1/1280 | 0,434 | 0,459 | 0,697 | 0,350 | 0,085 | 0,035 |
| 1/2560 | 0,298 | 0,350 | 0,590 | 0,325 | 0,080 | 0,034 |
| 1/5120 | 0,201 | 0,302 | 0,468 | 0,178 | 0,056 | 0,026 |
| 1/10240 | 0,156 | 0,189 | 0,363 | 0,110 | 0,027 | 0,021 |
| 1/20480 | 0,103 | 0,110 | 0,173 | 0,079 | - | - |
| 1/40960 | 0,089 | 0,071 | 0,142 | 0,055 | - | - |

[1], [2], [3], [4], [5] et [6] ont la même signification qu'au Tableau VI ci-dessus.

TABLEAU VIII (suite)

| DILUTIONS | P99-KLH a[6] | P99-KLH b[5] | P100 KLH[4] | P99-BSA[3] | TEMOIN P99[2] | TEMOIN P100[1] |
|---|---|---|---|---|---|---|
| 1/81920 | 0,058 | 0,046 | 0,111 | 0,053 | - | - |
| 1/163840 | 0,045 | 0,047 | 0,082 | 0,051 | - | - |
| 1/327680 | 0,033 | 0,046 | 0,062 | 0,050 | - | - |
| Sensibilisation des plaques ELISA avec : | P 99 libre | P 99 libre | P 100 libre | P 99 libre | P 100 libre | P 99 libre |

[1], [2], [3], [4], [5] et [6] ont la même signification qu'au Tableau VI ci-dessus.

La figure 5 représente les résultats obtenus au Tableau VIII ci-dessus, à savoir les titrations des deuxièmes sérums de lapins immunisés avec les peptides P99 et P100 conjugués à la KLH ou à la BSA, les plaques de microtitration étant sensibilisées avec les peptides P99 et P100 libres.

Cette figure comprend en ordonnée les densités optiques à 405 nm et en abscisse les dilutions des sérums (échelle log). Les courbes (1) et (2) correspondent aux témoins P99 et P100, la courbe (3) fournit les titrations d'un sérum de lapin immunisé avec le conjugué P99-BSA, la courbe (4) fournit les titrations d'un sérum de lapin immunisé avec le conjugué P100-KLH, les courbes (5) et (6) fournissent les titrations d'un sérum de lapin immunisé avec le conjugué P99-KLH.

**<u>EXEMPLE 10 :</u> Titrations des deuxièmes sérums (saignée après le deuxième rappel des mêmes lapins que ceux des Exemples 6 et 7) de lapins immunisés avec les peptides P99 et P100 conjugués à la KLH ou à la BSA, sensibilisés avec les peptides P99 et P100 conjugués.**

TABLEAU IX

| DILUTIONS | P99-BSA[3] | P99-KLH a[6] | P99 KLH b[5] | P100-KLH[4] | TEMOIN P99[2] | TEMOIN P100[1] |
|---|---|---|---|---|---|---|
| 1/10 | 0,835 | 0,804 | 1,057 | 0,859 | 0,492 | 0,292 |
| 1/20 | 0,861 | 0,879 | 0,950 | 0,881 | 0,488 | 0,258 |
| 1/40 | 0,829 | 0,902 | 0,970 | 0,731 | 0,520 | 0,205 |
| 1/80 | 0,825 | 0,924 | 0,980 | 0,856 | 0,330 | 0,189 |
| 1/160 | 0,789 | 0,960 | 1,021 | 0,836 | 0,258 | 0,112 |
| 1/320 | 0,785 | 0,904 | 0,956 | 0,779 | 0,201 | 0,088 |
| 1/640 | 0,658 | 0,879 | 0,837 | 0,669 | 0,196 | 0,066 |
| 1/1280 | 0,528 | 0,751 | 0,805 | 0,571 | 0,140 | 0,038 |
| 1/2560 | 0,415 | 0,735 | 0,742 | 0,510 | 0,098 | - |
| 1/5120 | 0,284 | 0,645 | 0,573 | 0,480 | 0,035 | - |
| 1/10240 | 0,249 | 0,482 | 0,568 | 0,350 | 0,020 | - |
| 1/20480 | 0,158 | 0,335 | 0,445 | 0,174 | - | - |
| 1/40960 | 0,101 | 0,184 | 0,425 | 0,092 | - | - |
| 1/81920 | 0,078 | 0,135 | 0,298 | 0,069 | - | - |
| 1/163840 | 0,115 | 0,102 | 0,245 | 0,065 | - | - |
| 1/327680 | 0,080 | 0,065 | 0,244 | 0,061 | - | - |
| Sensibilisation des plaques ELISA avec : | P99-KLH a[7] | P99-BSA[8] | P99-BSA[8] | P100-BSA[9] | P100-BSA[9] | P99-BSA[8] |

[1], [2], [3], [4], [5] et [6] ont la même signification qu'au Tableau VI ci-dessus.
[7], [8], et [9] ont la même signification qu'au Tableau VII ci-dessus.

La figure 6 représente les résultats obtenus au Tableau IX ci-dessus, à savoir les titrations des deuxièmes sérums de lapins immunisés avec les peptides P99 et P100 conjugués à la KLH ou à la BSA, les plaques de microtitration étant sensibilisées avec les peptides P99 et P100 conjugués.

Cette figure comprend en ordonnée les densités optiques à 405 nm et en abscisse les dilutions des sérums (échelle log). Les courbes (1) et (2) correspondent aux témoins P99 et P100, la courbe (3) fournit les titrations d'un sérum de lapin immunisé avec le conjugué P99-BSA, la courbe (4) fournit les titrations d'un sérum de lapin immunisé avec le conjugué P100-KLH et les courbes (5) et (6) fournissent les titrations d'un sérum de lapin immunisé avec le conjugué P99-KLH.

La figure 7 représente les résultats obtenus avec le peptide 102 ; la courbe (1) correspond au témoin P102 et la courbe (2) fournit les titrations d'un sérum de lapin immunisé avec le conjugué P102-KLH-gmb.

**EXEMPLE 11 : Préparation d'immunoconjugués comportant un anticorps antipeptide.**

De tels immunoconjugués ont comme cibles à la fois les cellules infectées par le VIF et le VIF lui-même.

a) Préparation de glucose oxydase conjuguée à un anticorps antipeptide conforme à l'invention :

Un anticorps polyclonal conforme à l'invention purifié à partir du sérum de lapin immunisé comme spécifié aux Exemples 6 à 10 ou un anticorps monoclonal conforme à l'invention, préparé de manière connue en soi, par immunisation d'un mammifère approprié, notamment et ce de manière non limitative, des souris Balb/c, avec un fragment peptidique conforme à l'invention, puis fusion des cellules spléniques avec des cellules myélomateuses appropriées conformément au protocole décrit par KOHLER et MILSTEIN, lequel anticorps monoclonal est couplé à de la glucose oxydase en utilisant du N-succinimidyl 3(2-pyridyl-dithio)propionate (SPDP) (PIERCE) et du 2-imino-thiolane (PIERCE) selon le protocole suivant :

a.1. Modification de la glucose oxydase :

La glucose oxydase (4 mg/ml) dans un tampon potassium phosphate 0,1 M (PB), pH 6,8 est mise en contact avec du SPDP en excès molaire d'un facteur 20. Après 30 min d'incubation à 30°C, l'échantillon est dialysé contre un tampon PB 0,1 M et de l'EDTA 1mM, pH 7,2. Dans les conditions ci-dessus, il n'y a pas de perte d'activité enzymatique.

a.2 : Modification de l'anticorps :

Un anticorps conforme à l'invention est mis en contact avec du 2-iminothiolane en excès molaire d'un facteur 250 pendant 1 heure à une température entre 0 et 4°C.

Pour retirer le 2-iminothiolane en excès, l'échantillon est passé sur une colonne "SEPHADEX" G25 équilibrée avec un tampon acétate de sodium 5 mM, NaCl 0,15 M et EDTA 1 mM, pH 5,2.

a.3. : La conjugaison est réalisée en mélangeant l'anticorps avec un poids égal d'enzyme modifiée.

Les solutions sont ensuite saturées avec de l'azote et incubées entre 40 et 70 heures à 4°C. On arrête la réaction par l'addition d'acide iodoacétique en excès molaire d'un facteur 100 et incubation pendant 30 min à 0°C.

Les conjugués sont séparés des protéines non couplées par une filtration sur gel FPLC "SUPEROSE-12" (10 $\mu$, 10 x 300 mm, Pharmacia) dans un tampon PBS.

b) Préparation de peroxydase conjuguée à un anticorps antipeptide conforme à l'invention :

On procède comme en a) à l'exception du fait que la peroxydase est modifiée en présence de SPDP en excès molaire d'un facteur 5.

c) Activité cytotoxique d'un mélange des deux immunoconjugués précités :

L'effet des immunotoxines en combinaison sur la viabilité des cellules infectées ou inoculées est étudié en utilisant trois tests différents : le test de libération $^{51}$Cr, le test colorimétrique MTT et la clonogénicité *in vitro.*

A titre d'exemple non limitatif, les exemples c.1, c.2 et c.3 montrent l'effet sur la viabilité cellulaire des immunotoxines anticorps-GO et anticorps-LPO selon l'Exemple 10 a) ou b), vis-à-vis de cellules lymphoides primaires infectées ou inoculées.

c.1 : <u>test de libération $^{51}$Cr</u>.

- Des cellules lymphoides primaires infectées ou inoculées sont cultivées *in vitro* dans du DMEM supplémenté avec 7 % de sérum de cheval décomplémenté.
- Les conjugués (anticorps-GO et anticorps-LPO) sont préparés conformément aux protocoles de l'Exemple 10 a) et b) :
- Test proprement dit : 1 à $5.10^6$ cellules infectées sont marquées avec 100 $\mu$Ci de $^{51}$chromate de sodium dans du DMEM-HS 10 % pendant une heure à 37°C dans un incubateur à $CO_2$ humide. Environ 1 500 à 4 500 cpm sont incorporés /$10^4$ cellules. Les cellules sont lavées et incubées pendant 20 min à 37°C dans du sérum de lapin normal décomplémenté (RNS), dans un milieu à raison de $10.10^6$ cellules/ml dans des tubes de Falcon n° 2 001, comprenant une solution saline équilibrée de Dulbecco tamponnée au bicarbonate et du HS à 2,5 % décomplémenté (BBSS-HS 2,5 %). Les cellules sont lavées et 50 µl ($1.10^5$ cellules) sont distribuées dans les puits d'une microplaque, puis on incorpore 50 µl de différentes dilutions des immunotoxines (anticorps-GO mélangé avec anticorps-LPO), lesdites dilutions étant réalisées dans du BSS-HS 2,5 %. Après 20 min d'incubation à 22°C, suivie de deux lavages, les cellules sont mises en suspension dans 100 µl de milieu complet (CM) : BBSS-HS ou -FBS 2,5 % supplémenté avec 4 g/l de dextrose et 0,1 mM NaI, transférées dans des tubes Falcon n° 2054 et placées à 37°C dans un incubateur à $CO_2$ humide pour réaliser la réaction cytotoxique. Les tubes sont retirés à des moments différents, centrifugés et la radioactivité libérée est déterminée sur un échantillon de surnageant de 50 µl. Le pourcentage de $^{51}Cr$ libéré est déterminé à partir de la formule suivante :

$$\frac{\text{coups/min ds surnag. (50 µl) - coups/min B.F.}^{(*)}}{\text{coups/min ds surnag.(cell. mises en susp. ds sol. HCl 4N) - coups/min B.F.}} \text{x100}$$

(*) B.F. = bruit de fond.
et correspond au pourcentage de coups/min relargués spécifiquement.

c.2 : <u>essai MTT</u>

Des cellules lymphoides primaires infectées ou inoculées sont incubées dans des microplaques de microculture à une concentration de $1.10^6$/ml avec différentes dilutions d'anticorps-GO et d'anticorps-LPO, lesdites dilutions étant réalisées dans du BSS-HS 2,5 % ou du BSS-HS 2,5 % tamponné Hepes (HBSS) ou du BSS-FBS 2,5 %. On utilise comme contrôle des cellules lymphoides primaires incubées avec des immunotoxines ne réagissant pas avec lesdites cellules. Après 20 min à 22°C, puis lavage, $1.10^5$ cellules sont transférées sur d'autres microplaques et mises en suspension dans 100 µl de CM et placées à 37°C dans un incubateur à $CO_2$ humide. Après 45 min à deux heures d'incubation à 37°C, les cellules sont lavées dans du DMEM-HS 2,5 %, mises en suspension dans 100 µl de DMEM-HS 2,5 % et 10 µl de colorant MTT tetrazolium (5 mg/ml) est ajouté à chaque puits. Après une nouvelle incubation de deux heures à 37°C, les microplaques sont centrifugées et 100 µl de DMSO sont ajoutés à chaque puits. La couleur développée est estimée spectrophotométriquement à 540 nm dans un appareil approprié. La densité optique des cellules infectées non-traitées (100 % de viabilité) est comprise entre 0,8 et 1,4 de densité optique. Les résultats sont exprimés comme le pourcentage de l'absorbance-contrôle comme suit :

$$\frac{\text{DO des cellules traitées - DO de la ligne de base}}{\text{DO des cellules non-traitées - DO de la ligne de base}} \text{x100}$$

c.3 : <u>essai de clonogénicité</u>

Des cellules lymphoides primaires Infectées ou inoculées sont incubées comme décrit dans l'exemple c. 1 (test au $^{51}Cr$), la concentration cellulaire étant de $0,6.10^6$/ml. Après une incubation de 20 min à deux heures à 37°C dans du CM, les cellules sont lavées deux fois, mises en suspension dans du DMEM-HS 10 % et sont distribuées à des concentrations de $10^4$, $10^3$, $10^2$ et 10 cellules par puits dans des plaques de microculture à fond plat comprenant 96 puits. Les plaques sont cultivées à 37°C pendant 6 jours ; au sixième jour les cellules sont supplémentées avec 100 µl de milieu frais et au douzième jour de 100 µl de milieu est retiré et est remplacé par 100 µl de milieu frais. Les microplaques sont observées au microscope et les résultats sont exprimés par la fréquence de cellules survivantes.

Le nombre de cellules lymphoides primaires qui ne sont pas détruites est estimé au moyen du test de clonogénicité.

Ainsi que cela ressort de ce qui précède, l'invention ne se limite nullement à ceux de ses modes de mise en oeuvre, de réalisation et d'application qui viennent d'être décrits de façon plus explicite ; elle en embrasse au contraire toutes les variantes qui peuvent venir à l'esprit du technicien en la matière, sans s'écarter du cadre, ni de la portée, de la présente invention.

## Revendications

1. Fragment peptidique comprenant une portion d'une protéine *env* du virus de l'immunodéficience féline (VIF) comprise entre l'aminoacide 66 et l'aminoacide C terminal de la protéine *env,* caractérisé en ce qu'il est sélectionné dans le groupe constitué par :

- un fragment peptidique qui comprend 19 amino-acides, contient au moins un épitope immunogène majeur de ladite protéine *env* du VIF et présente la formule I suivante :

  Asn-Gln-Thr-Cys-Met-Trp-Asn-Thr-Ser-Gln-Ile-Gln-Asp-Pro-Glu-Ile-Pro-Lys-Cys. (I),

  qui correspond aux positions 336-355 de la protéine *env* de la souche *Petaluma,*
- un fragment peptidique qui comprend 21 aminoacides, contient au moins un épitope immunogène majeur de ladite protéine *env* du VIF et présente la formule II suivante :

  Leu-Val-Val-Pro-Glu-Glu-Val-Met-Glu-Tyr-Lys-Pro-Arg-Arg-Lys-Arg-Ala-Ala-Ile-His-Val. (II),

  qui correspond aux positions 596-616 de la protéine *env* de la souche *Petaluma* et dans laquelle X représente Leu ou Val,
- un fragment peptidique qui comprend 25 aminoacides, contient au moins un épitope immunogène majeur de ladite protéine *env* du VIF et présente la formule III suivante :

  Val-Ile-Ala-Met-Pro-Glu-Val-Glu-Gly-Glu-Glu-Ile-Gln-Pro-Gln-Met-Glu-Leu-Arg-Arg-Asn-Gly-Arg-Gln-Cys (III),

  qui correspond aux positions 824-848 de la protéine *env* de la souche *Petaluma,*
- un fragment peptidique qui comprend 26 amino-acides et présente la formule IV suivante :

  Leu-Arg-Asn-Glu-Ile-Gln-Glu-Val-Lys-Leu-Glu-Glu-Gly-Asn-Ala-Gly-Lys-Phe-Arg-Arg-Ala-Arg-Phe-Leu-Arg-Tyr (IV),

  qui correspond aux positions 66-91 de la protéine *env* de la souche *Petaluma,* et
- un fragment peptidique qui comprend 28 amino-acides et présente la formule V suivante :

  Leu-Gln-Gly-Lys-Ile-Asn-Ile-Ser-Leu-Cys-Leu-Thr-Gly-Gly-Lys-Met-Leu-Tyr-Asn-Lys-Val-Thr-Lys-Gln-Leu-Ser-Tyr-Cys (V),

  qui correspond aux positions 292-320 de la protéine *env* de la souche *Petaluma,*

lesquels fragments sont reconnus au moins par les anticorps produits lors d'une infection et/ou lors d'une inoculation par l'une quelconque des souches de VIF.

**2.** Anticorps anti-fragment peptidique, caractérisés en ce qu'ils sont obtenus par immunisation d'un animal approprié avec au moins un fragment peptidique selon la revendication 1, lesquels anticorps présentent une affinité pour ledit peptide, et sont aptes à reconnaître la protéine *env* du VIF, le VIF et/ou les cellules exprimant le VIF.

**3.** Anticorps selon la revendication 2, caractérisés en ce que le fragment peptidique est sélectionné dans le groupe constitué par le fragment peptidique de formule I, le fragment peptidique de formule II et le fragment peptidique de formule III.

**4.** Réactif immunologique utilisable pour la détection, le diagnostic et le suivi de l'immunodéficience féline, caractérisé en ce qu'il est choisi dans le groupe constitué par les fragments peptidiques selon la revendication 1 et les anticorps anti-peptide selon la revendication 2, éventuellement associés à une autre substance, notamment une enzyme.

**5.** Réactif selon la revendication 4, caractérisé en ce qu'il est constitué par un mélange des peptides selon la revendications 1.

**6.** Procédé de détection et/ou de diagnostic de l'immunodéficience féline, caractérisé en ce qu'il consiste à détecter les anticorps éventuellement présents dans un fluide biologique tel que le sang, en mettant en présence ledit fluide avec au moins un fragment peptidique selon la revendication 1, auquel se lient les anticorps anti-VIF, si de tels anticorps sont présents dans l'échantillon biologique à contrôler, la lecture du résultat étant révélé par un moyen approprié, notamment RIA, EIA ou cytométrie en flux.

**7.** Kit ou trousse de diagnostic pour la détection, le diagnostic ou le suivi de l'immunodéficience féline, caractérisé en ce qu'il comprend :

- un support solide approprié revêtu d'au moins un fragment peptidique selon la revendication 1,
- des quantités ou doses appropriées de conjugué choisi dans le groupe qui comprend les conjugués enzyme appropriée-anticorps anti-Ig félins appropriés et les conjugués enzyme appropriée-anticorps anti-peptide selon la revendication 2 ou la revendication 3,
- des quantités ou doses appropriées d'une substance de révélation convenable.

**8.** Procédé d'identification de souche de VIF, caractérisé en ce que l'on met en contact un échantillon à analyser, notamment un fluide biologique avec un panel de fragments peptidiques selon la revendication 1, et en ce que l'on détermine par tout moyen approprié le/les fragments peptidiques qui se sont éventuellement liés aux anticorps produits par la souche à détecter.

**9.** Agents à visées thérapeutiques et/ou préventives, caractérisés en ce qu'ils sont constitués par, ou comprennent en tant que constituant actif, un peptide selon la revendication 1, seul ou conjugué ou associé à d'autres substances.

**10.** Agents à visée thérapeutique et/ou préventive, caractérisés en ce qu'ils sont constitués par, ou comprennent en tant que constituants actifs, des anticorps anti-peptide selon la revendication 2 ou la revendication 3, seuls ou conjugués avec d'autres substances.

**11.** Agents selon la revendication 10, caractérisés en ce qu'ils sont associés à une enzyme oxydative.

**12.** Agents selon la revendication 10 ou la revendication 11, caractérisés en ce que l'enzyme oxydative est choisie dans le groupe qui comprend les enzymes oxydatives générant de l'oxygène actif et les enzymes oxydatives dont le substrat est de l'oxygène actif et un halogénure.

**13.** Agents selon l'une quelconque des revendications 10 à 12, caractérisés en ce que les enzymes oxydatives générant de l'oxygène actif sont choisies dans le groupe qui comprend la glucose oxydase (GO), la NADPH oxydase, la xanthine oxydase, notamment la xanthine oxydase du lait, la cholestérol oxydase, la choline oxydase, l'acide lactique oxydase, la pyruvate oxydase.

**14.** Agents selon l'une quelconque des revendications 10 à 12, caractérisés en ce que les enzymes oxydatives dont le substrat est un halogénure sont choisies dans le groupe qui comprend la lactoperoxydase (LPO), la myéloperoxydase, notamment la myéloperoxydase des neutrophiles, la peroxydase des éosinophiles, et la peroxydase salivaire.

**15.** Méthode d'évaluation de l'efficacité d'immunoconjugués, caractérisé en ce qu'elle comprend :

- des cellules de chat aptes à développer le VIF et/ou infectées ou inoculées par ledit virus, et
- des immunoconjugués comprenant des enzymes oxydatives couplées à des anticorps choisis dans le groupe qui comprend les anticorps anti-VIF et les anticorps anti-peptide selon la revendication 2 ou la revendication 3.

**16.** Méthode d'évaluation selon la revendication 15, caractérisé en ce que les cellules aptes à développer le VIF et/ ou infectées ou inoculées par ledit virus sont notamment des cellules mononucléaires de chats infectés, des cellules lymphoïdes primaires et des lignées lymphoblastiques T félines, notamment les lignées MYA-1.

**17.** Procédé d'évaluation de l'efficacité d'immunoconjugués, caractérisé en ce que des immunoconjugués comprenant des enzymes oxydatives couplées à un anticorps approprié choisi dans le groupe qui comprend les anticorps anti-VIF et les anticorps anti-peptide selon la revendication 2 ou la revendication 3 sont mis en contact avec des cellules de chat aptes à développer le VIF et/ou infectées ou inoculées par ledit virus et en ce que la toxicité desdits immunoconjugués vis-à-vis desdites cellules, dites cellules cibles, est mesurée par tout moyen approprié.

**18.** Application de la méthode d'évaluation selon la revendication 16 au virus de l'immunodéficience humaine.

**19.** Application du procédé d'évaluation selon la revendication 17 au virus de l'immunodéficience humaine.

**Patentansprüche**

**1.** Peptidfragment, umfassend einen Teil eines env-Proteins des Katzenimmundefekt-Virus (VIF), der den Bereich zwischen Aminosäure 66 und der C-terminalen Aminosäure des env-Proteins umfaßt, dadurch **gekennzeichnet**, daß es aus der folgenden Gruppe ausgewählt ist:

- einem Peptidfragment, das 19 Aminosäuren umfaßt, mindestens ein hauptsächliches immunogenes Epitop des env-Proteins des VIF enthält und die folgende Formel (I):

  Asn-Gln-Thr-Cys-Met-Trp-Asn-Thr-Ser-Gln-Ile-Gln-Asp-Pro-
  Glu-Ile-Pro-Lys-Cys (I),

  besitzt, die den Positionen 336-355 des *env*-Proteins des Stamms *Petaluma* entspricht,

- einem Peptidfragment, das 21 Aminosäuren umfaßt, mindestens ein hauptsächliches immunogenes Epitop des env-Proteins von VIF enthält und folgende Formel (II):

  Leu-Val-Val-Pro-Glu-Glu-Val-Met-Glu-Tyr-Lys-Pro-Arg-Arg-
  Lys-Arg-Ala-Ala-Ile-His-Val (II),

  besitzt, die den Positionen 596-616 des *env*-Proteins des Stamms *Petaluma* entspricht und worin X Leu oder Val bedeutet,

- einem Peptidfragment, das 25 Aminosäuren umfaßt, mindestens ein hauptsächliches immunogenes Epitop des env-Proteins von VIF enhält und die folgende Formel (III):

  Val-Ile-Ala-Met-Pro-Glu-Val-Glu-Gly-Glu-Glu-Ile-Gln-Pro-
  Gln-Met-Glu-Leu-Arg-Arg-Asn-Gly-Arg-Gln-Cys (III),

  besitzt, die den Positionen 824-848 des *env*-Proteins des Stamms *Petaluma* entspricht,

- einem Peptidfragment, daß 26 Aminosäuren umfaßt und die folgende Formel (IV):

  Leu-Arg-Asn-Glu-Ile-Gln-Glu-Val-Lys-Leu-Glu-Glu-Gly-Asn-
  Ala-Gly-Lys-Phe-Arg-Arg-Ala-Arg-Phe-Leu-Arg-Tyr (IV),

  besitzt, die den Positionen 66-91 des *env*-Proteins des Stamms *Petaluma* entspricht, und

- einem Peptidfragment, das 28 Aminosäuren umfaßt und die folgende Formel (V):

Leu-Gln-Gly-Lys-Ile-Asn-Ile-Ser-Leu-Cys-Leu-Thr-Gly-Gly-
Lys-Met-Leu-Tyr-Asn-Lys-Val-Thr-Lys-Gln-Leu-Ser-Tyr-Cys
(V)

besitzt, die den Position 292-320 des *env*-Proteins des Stamms *Petaluma* entspricht,

wobei die Fragemente mindestens von den während einer Infektion und/oder während einer Inokulation mit einem der VIF-Stämme gebildeten Antikörper erkannt werden.

**2.** Anti-Peptidfragment-Antikörper, dadurch **gekennzeichnet**, daß sie durch Immunisierung eines geeigneten Tiers mit mindestens einem Peptidfragment nach Anspruch 1 erhalten werden, wobei die Antikörper eine Affinität für das Peptid besitzen und das env-Protein von VIF, VIF und/oder das VIF exprimierende Zellen erkennen können.

**3.** Antikörper nach Anspruch 2, dadurch **gekennzeichnet**, daß das Peptidfragment aus der Gruppe, bestehend aus dem Peptidfragment der Formel (I), dem Peptidfragment der Formel (II) und dem Peptidfragment der Formel (III) ausgewählt ist.

**4.** Immunologisches Reagens, das zum Nachweis, zur Diagnostik und der Verlaufskontrolle eines Immundefekts bei Katzen verwendet werden kann, dadurch **gekennzeichnet**, daß es aus der Gruppe, bestehed aus den Peptidfragmenten nach Anspruch 1 und den Anti-Peptid-Antikörpern nach Anspruch 2 ausgewählt ist, gegebenenfalls zusammen mit einer weiteren Substanz, insbesondere einem Enzym.

**5.** Reagens nach Anspruch 4, dadurch **gekennzeichnet**, daß es aus einem Gemisch der Peptide nach Anspruch 1 besteht.

**6.** Verfahren zum Nachweis und/oder zur Diagnostik eines Immundefekts bei Katzen, dadurch **gekennzeichnet**, daß man die in einer biologischen Flüssigkeit, wie Blut, gegebenenfalls vorhandenen Antikörper nachweist, indem man die Flüssigkeit mit mindestens einem Peptidfragment nach Anspruch 1 in Kontakt bringt, an das sich die Anti-VIF-Antikörper binden, wenn derartige Antikörper in der zu kontrollierenden biologischen Probe vorhanden sind, und das Ergebnis das mit einem geeigneten Mittel, insbesondere einem RIA, EIA oder Durchflußzytometrie gewonnen wurde, abliest.

**7.** Kit oder Diagnostikzusammenstellung zum Nachweis, zur Diagnose oder zur Verlaufskontrolle eines Immundefekts bei der Katze, dadurch **gekennzeichnet**, daß es umfaßt:

- einen festen Träger, der mit mindestens einem Peptidfragment nach Anspruch 1 beschichtet werden kann,
- geeignete Mengen oder Dosen des Konjugats, ausgewählt aus der Gruppe, umfassend Konjugate aus einem geeigneten Enzym und geeigneten Anti-Ig-Katzenantikörpern und den Konjugaten aus geeigneten Enzymen und Anti-Peptid-Antikörpern nach Anspruch 2 oder 3,
- geeignete Mengen oder Dosen einer geeigneten Nachweissubstanz.

**8.** Verfahren zur Identifikation des VIF-Stamms, dadurch **gekennzeichnet**, daß man eine zu analysierende Probe, insbesondere eine biologische Flüssigkeit, mit einer Reihe von Peptidfragmenten nach Anspruch 1 in Kontakt bringt, und daß man mit jedem geeigneten Mittel das/die Peptidfragment(e) bestimmt, das/die gegebenenfalls an die von dem nachzuweisenden Stamm gebildeten Antikörper gebunden ist/sind.

**9.** Mittel zur therapeutischen und/oder präventiven Verwendung, dadurch **gekennzeichnet**, daß sie aus einem Peptid nach Anspruch 1, allein oder konjugiert oder assoziiert mit anderen Substanzen, als Wirkstoff bestehen oder dieses umfassen.

**10.** Mittel zur therapeutischen und/oder präventiven Verwendung, dadurch **gekennzeichnet**, daß sie aus Anti-Peptid-Antikörpern nach Anspruch 2 oder 3 allein oder in Konjugation mit anderen Substanzen bestehen oder diese umfassen.

11. Mittel nach Anspruch 10, dadurch **gekennzeichnet**, daß sie mit einem oxidativen Enzym assoziiert sind.

12. Mittel nach Anspruch 10 oder 11 dadurch **gekennzeichnet**, daß das oxidative Enzym aus der Gruppe, umfassend oxidative Enzyme, die aktiven Sauerstoff erzeugen, und oxidative Enzyme, deren Substrat aktiver Sauerstoff und ein Halogenid ist, ausgewählt sind.

13. Mittel nach einem der Ansprüche 10 bis 12, dadurch **gekennzeichnet**, daß die oxidativen Enzyme, die aktiven Sauerstoff erzeugen, aus der Gruppe, umfassend Glucoseoxidase (GO), NADPH-Oxidase, Xanthinoxidase, insbesondere Milch-Xanthinoxidase, Cholesterinoxidase, Cholinoxidase, Milchsäureoxidase, Pyruvatoxidase, ausgewählt sind.

14. Mittel nach einem der Ansprüche 10 bis 12, dadurch **gekennzeichnet**, daß die oxidativen Enzyme, deren Substrat ein Halogenid ist, aus der Gruppe, umfassend Lactoperoxidase (LPO), Myeloperoxidase, insbesondere Neutrophilen-Myeloperoxidase, Eosinophilen-Peroxidase und Speichel-Peroxidase, ausgewählt sind.

15. Verfahren zur Bewertung der Wirksamkeit eines Immunkonjugats, dadurch **gekennzeichnet**, daß es umfaßt:

- Katzenzellen, die geeignet sind, VIF zu bilden und/oder mit dem Virus infiziert oder inokuliert sind, und

- Immunkonjugate, umfassend oxidative Enzyme, die an Antikörper gekoppelt sind, die aus der Gruppe, umfassend Anti-VIF-Antikörper und Anti-Peptid-Antikörper, nach Anspruch 2 oder 3 ausgewählt sind.

16. Verfahren zur Bewertung nach Anspruch 15, dadurch **gekennzeichnet**, daß die Zellen, die geeignet sind VIF zu bilden und/oder mit dem Virus infiziert oder inokkuliert sind, insbesondere mononukleare Zellen der infizierten Katze, primäre lymphoide Zellen und T-Lymphoblasten-Zelllinien der Katze, insbesondere MYA-1-Linien, sind.

17. Verfahren zur Bewertung der Wirksamkeit der Immunkonjugate, dadurch **gekennzeichnet**, daß die Immunkonjugate oxidative Enzyme gekoppelt an einen geeigneten Antikörper, ausgewählt aus der Gruppe, umfassend Anti-VIF-Antikörper und Anti-Peptidantikörper nach Anspruch 2 oder 3 umfassen, in Kontakt mit den Zellen der Katze gebracht werden, die geeignet sind VIF zu bilden und/oder mit dem Virus infiziert oder inokuliert sind, und daß die Toxizität der Immunkonjugate gegenüber der Zellen, d.h. der Zielzellen, mit jedem geeigneten Mittel gemessen wird.

18. Verwendung des Verfahrens zur Bewertung nach Anspruch 16 auf das menschliche Immundefektvirus.

19. Verwendung des Verfahrens zur Bewertung nach Anspruch 17 auf das menschliche Immundefektvirus.

**Claims**

1. A peptidic fragment comprising a portion of an env protein of the feline immunodeficiency virus (FIV) comprised between the amino acid 66 and the C-terminal amino acid of env protein, characterized in that it is selected from the group constituted by:

- a peptidic fragment which comprises 19 amino acids, contains at least one major immunogenic epitope of the said env protein of the FIV and has the following formula I:

Asn-Gln-Thr-Cys-Met-Trp-Asn-Thr-Ser-Gln-Ile-Gln-Asp-Pro-
Glu-Ile-Pro-Lys-Cys, (I)

which corresponds to positions 336-355 of the env protein of the Petaluma strain,
- a peptidic fragment which comprises 21 amino acids, contains at least one major immunogenic epitope of the said env protein of the FIV and has the following formula II:

Leu-Val-Val-Pro-Glu-Glu-Val-Met-Glu-Tyr-Lys-Pro-Arg-Arg-
Lys-Arg-Ala-Ala-Ile-His-Val, (II)

which corresponds to the positions 596-616 of the env protein of the Petaluma strain and wherein X is Leu or Val,

- a peptidic fragment which comprises 25 amino acids, contains at least one major immunogenic epitope of the said env protein of the FIV and has the following formula III:

Val-Ile-Ala-Met-Pro-Glu-Val-Glu-Gly-Glu-Glu-Ile-Gln-Pro-
Gln-Met-Glu-Leu-Arg-Arg-Asn-Gly-Arg-Gln-Cys (III)

which corresponds to the positions 824-848 of the env protein of the Petaluma strain,

- a peptidic fragment which comprises 26 amino acids and has the following formula IV:

Leu-Arg-Asn-Glu-Ile-Gln-Glu-Val-Lys-Leu-Glu-Glu-Gly-Asn-
Ala-Gly-Lys-Phe-Arg-Arg-Ala-Arg-Phe-Leu-Arg-Tyr (IV)

which corresponds to the positions 66-91 of the env protein of the Petalum strain, and

- A peptidic fragment which comprises 28 amino acids and has the following formula V:

Leu-Gln-Gly-Lys-Ile-Asn-Ile-Ser-Leu-Cys-Leu-Thr-Gly-Gly-
Lys-Met-Leu-Tyr-Asn-Lys-Val-Thr-Lys-Gln-Leu-Ser-Tyr-Cys (V)

which corresponds to the positions 292-320 of the env protein of the Petaluma strain,

which fragments are recognized at least by the antibodies produced at the time of an infection and/or at the time of an inoculation by any one of the FIV strains.

**2.** Anti-peptidic fragment antibodies, characterized in that they are obtained by the immunization of a suitable animal with at least one peptidic fragment according to claim 1, which antibodies have an affinity to the said peptide, and are able to recognized the env protein of the FIV, the FIV and/or the cells expressing the FIV.

**3.** Antibodies according to claim 2, characterized in that the peptidic fragment is selected within the group constituted by the peptidic fragment of formula I, the peptidic fragment of formula II and the peptidic fragment of formula III.

**4.** An immunologic reagent usable for the detection, the diagnostic and the follow-up of the feline immunodeficiency, characterized in that it is selected from the group constituted by the peptidic fragments of claim 1 and the anti-peptide antibodies of claim 2, possibly associated with another substance, particularly an enzyme.

**5.** A reagent according to claim 4, characterized in that it is constituted by a mixture of the peptides according to claim 1.

**6.** A process for the detection and/or diagnosis of feline immunodeficiency, characterized in that it consists in detecting any antibodies that may be present in a biological fluid such as blood, by bringing together the said fluid and at least one peptidic fragment according to claim 1, to which the anti-FIV antibodies become bound, if such antibodies are present in the biological sample to be monitored, the reading of the result being revealed by suitable means, particularly RIA, EIA or flux cytometry.

**7.** A diagnosis kit or instrument case for the detection, the diagnosis or the follow-up of feline immunodeficiency, characterized in that it comprises:

- a suitable solid support coated with at least one peptidic fragment according to claim 1,
- suitable amounts or doses of conjugate selected from the group which comprises the conjugates of suitable enzyme-suitable feline anti-Ig antibodies and the conjugates of suitable enzyme-anti-peptide antibodies according to claim 2 or claim 3,
- suitable amounts or doses of an appropriate disclosure substance.

**8.** A process for the identification of a FIV strain, characterized in that contact is established between a sample to be analyzed, particularly a biological fluid, with a panel of peptitidic fragments according to claim 1, and in that the determination is made by any suitable means of the peptidic fragment(s) which may have become bound with the antibodies produced by the strain to be detected.

**9.** Agents intended for treatment and/or prevention, characterized in that they are constituted by, or contain as the active constituent, a peptide according to claim 1, alone or conjugated or associated with other substances.

**10.** Agents intended for treatment and/or prevention, characterized in that they are constituted by, or contain as active constituents, anti-peptide antibodies according to claim 2 or claim 3, aldne or conjugated with other substances.

**11.** Agents according to claim 10, characterized in that they are associated with an oxidative enzyme.

**12.** Agents according to claim 10 or claim 11, characterized in that the oxidative enzyme is selected from the group which comprises oxidative enzymes generating active oxygen and oxidative enzymes the substrate of which is active oxygen and a halide.

**13.** Agents according to any one of claims 10 to 12, characterized in that the oxidative enzymes generating active oxygen are selected from the group which comprises glucose oxidase (GO), NADPH oxidase, xanthine oxidase, particularly milk xanthine oxidase, cholesterol oxidase, choline oxidase, lactic acid oxidase, pyruvate oxidase.

**14.** Agents according to any one of claims 10 to 12, characterized in that the oxidative enzymes the substrate of which is a halide are selected from the group which contains lactoperoxidase (LPO), myeloperoxidase, particularly myeloperoxidase of neutrophiles, peroxidase of eosinophiles, and salivary peroxidase.

**15.** A method of evaluation of the efficiency of immunoconjugates, characterized in that it comprises:

- cat cells able to develop the FIV and/or infected or inoculated by the said virus, and
- immunoconjugates comprising oxidative enzymes coupled with antibodies chosen from the group which comprises anti-FIV antibodies and anti-peptide antibodies according to claim 2 or claim 3.

**16.** A method of evaluation according to claim 15, characterized in that the cells able to develop the FIV and/or infected or inoculated by the said virus are in particular mononuclear cells of infected cats, primary lymphoid cells and feline T lymphoblastic lines, particularly the MYA-1 lines.

**17.** A method of evaluation of the efficiency of immunoconjugates, characterized in that immunoconjugates comprising oxidative enzymes coupled with a suitable antibody chosen from the group which comprises anti-FIV antibodies and anti-peptide antibodies according to claim 2 or claim 3 are placed in confact with cat cells able to develop FIV and/or infected or inoculated by the said virus, and in that the toxicity of the said immunoconjugates with respect to the said cells, termed target cells, is measured by any suitable means.

**18.** Application of the method of evaluation according to claim 16 to the virus of human immunodeficiency.

**19.** Application of the method of evaluation according to claim 17 to the virus of human immunodeficiency.

| | | |
|---|---|---|
| Peptide 99 | NQTCMWNTSQIQDPEIPKC | (SU, 336-355) |
| Peptide 100 | LVVPEEVMEYKPRRKRAAIHV | (SU, 596-616) |
| Peptide 101 | LRNEIQEVKLEEGNAGKFRRARFLRY | (SU, 66-91) |
| Peptide 102 | VIAMPEVEGEEIQPQMELRRNGRQC | (SU., 824-848) |
| Peptide 103 | LQGKINISLCLTGGKMLYNKVTKQLSYC | (SU, 292-320) |

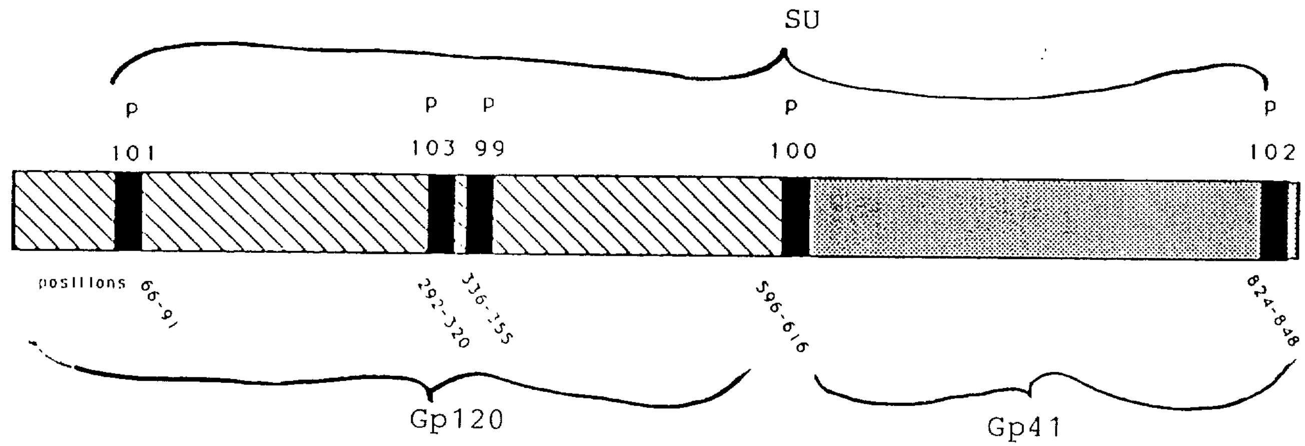


FIGURE 1

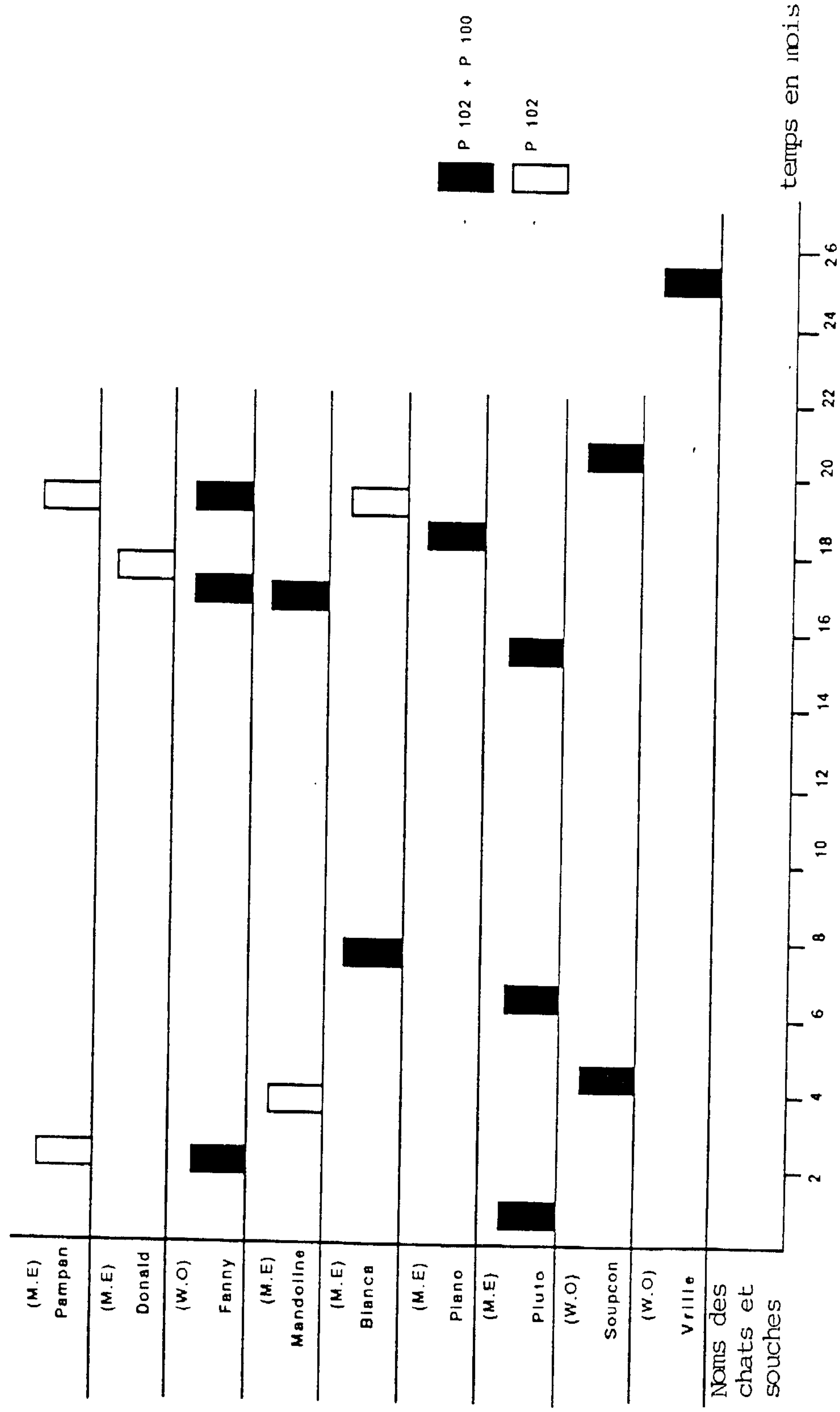
(M.E) Pampan
(M.E) Donald
(W.O) Fanny
(M.E) Mandoline
(M.E) Bianca
(M.E) Piano
(M.E) Pluto
(W.O) Soupcon
(W.O) Vrille
Noms des chats et souches
2
4
6
8
10
12
14
16
18
20
22
24
26
temps en mois
P 102 + P 100
P 102

FIGURE 2

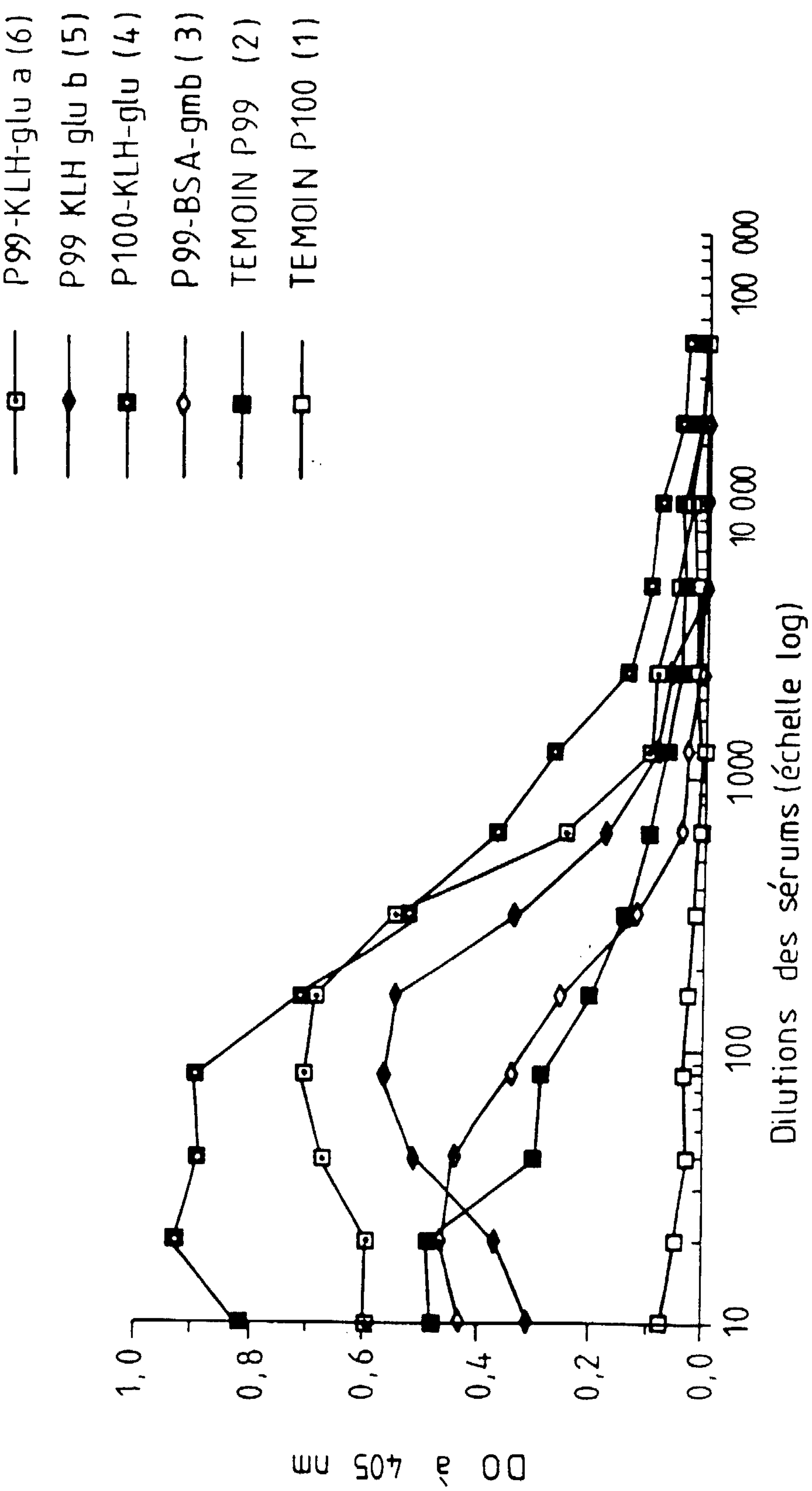
P99-KLH-glu a (6)
P99 KLH glu b (5)
P100-KLH-glu (4)
P99-BSA-gmb (3)
TEMOIN P99 (2)
TEMOIN P100 (1)
DO à 405 nm
1,0
0,8
0,6
0,4
0,2
0,0
10
100
1000
10 000
100 000
Dilutions des sérums (échelle log)

FIG. 3

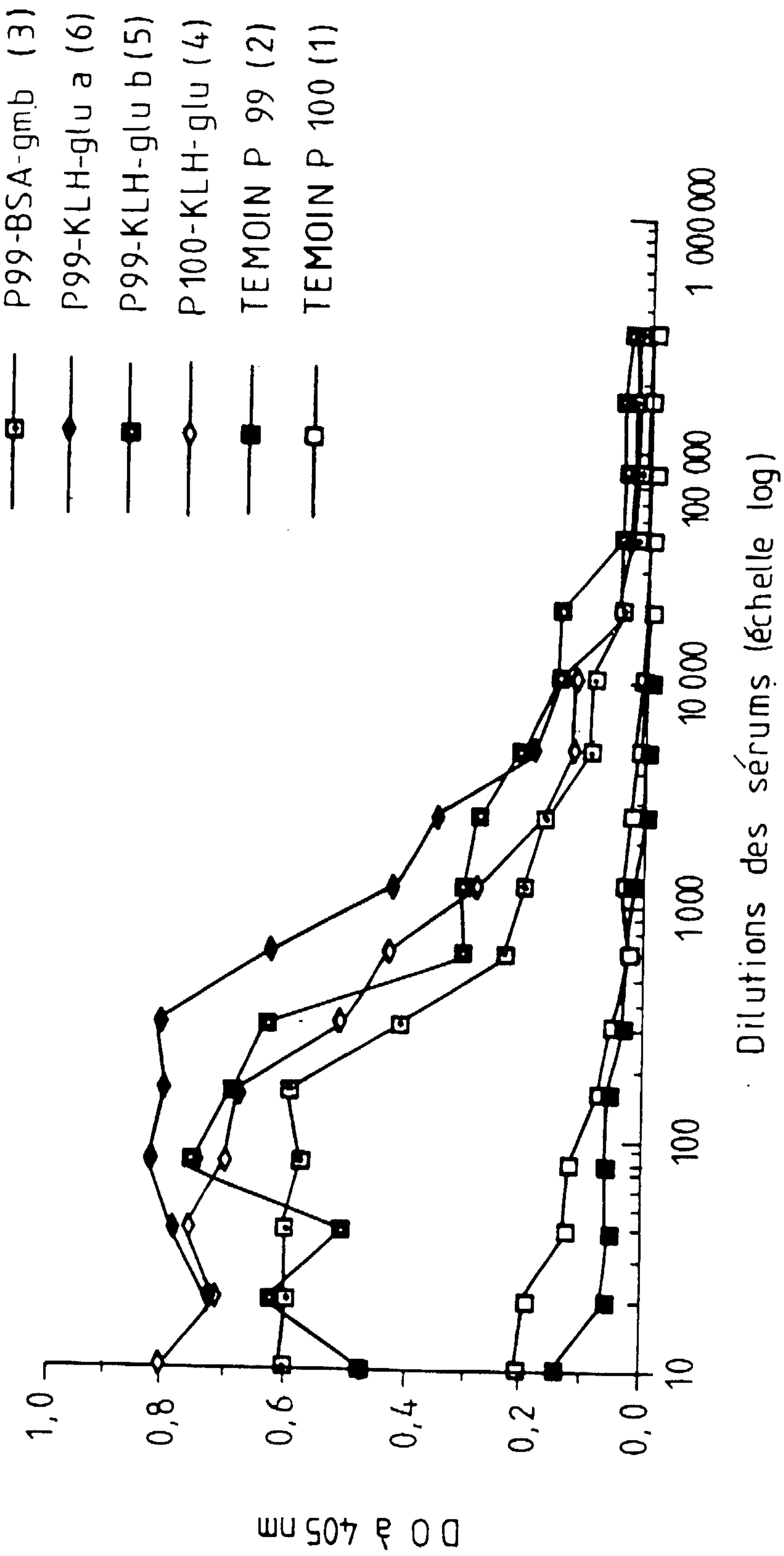
P99-BSA-gmb (3)
P99-KLH-glu a (6)
P99-KLH-glu b (5)
P100-KLH-glu (4)
TEMOIN P 99 (2)
TEMOIN P 100 (1)
1,0
0,8
0,6
0,4
0,2
0,0
10
100
1000
10 000
100 000
1 000 000
Dilutions des sérums (échelle log)
D O à 405 nm

FIG.4

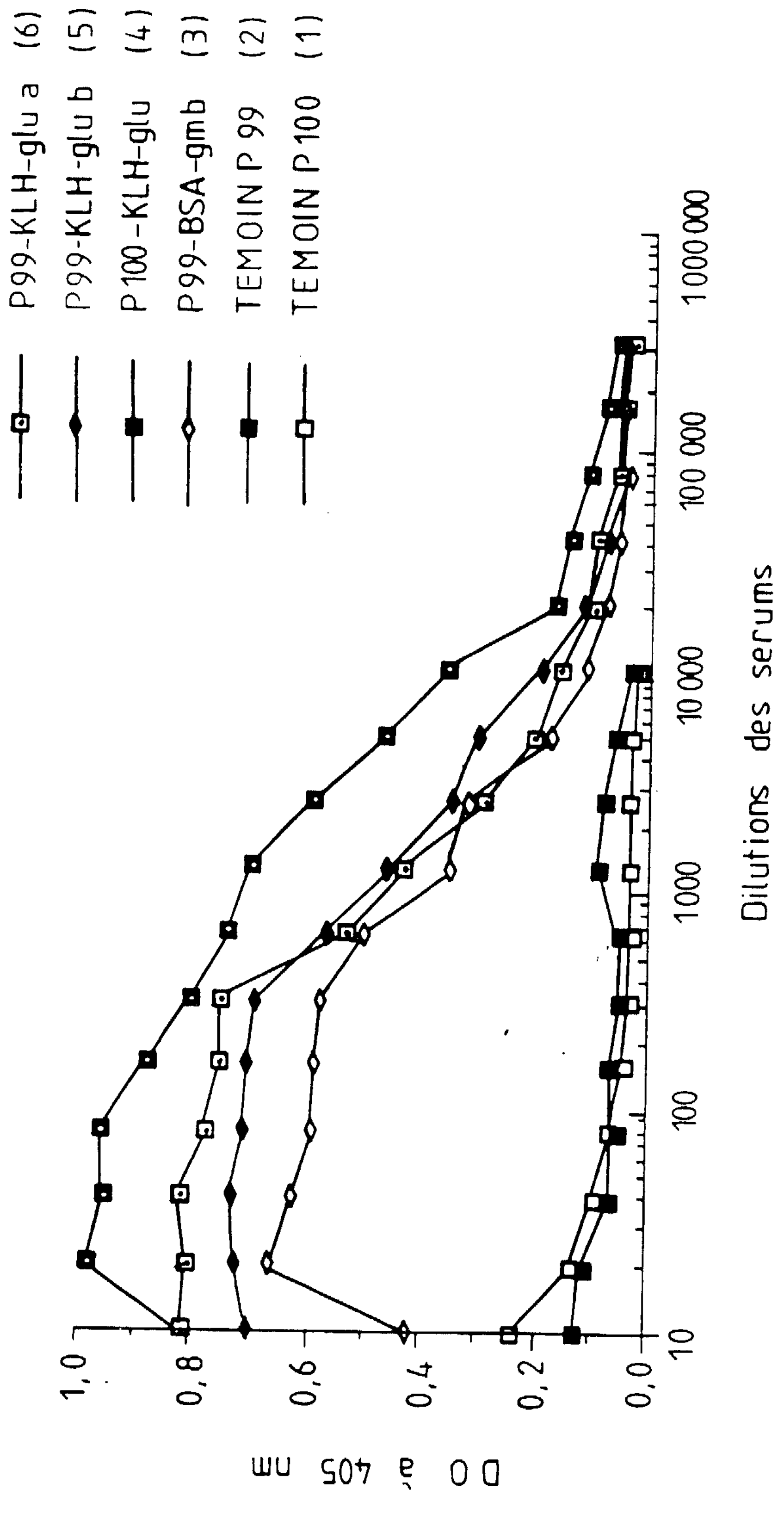
P99-KLH-glu a (6)
P99-KLH-glu b (5)
P100-KLH-glu (4)
P99-BSA-gmb (3)
TEMOIN P 99 (2)
TEMOIN P 100 (1)
D O à 405 nm
1,0
0,8
0,6
0,4
0,2
0,0
10
100
1000
10 000
100 000
1000 000
Dilutions des serums

FIG.5

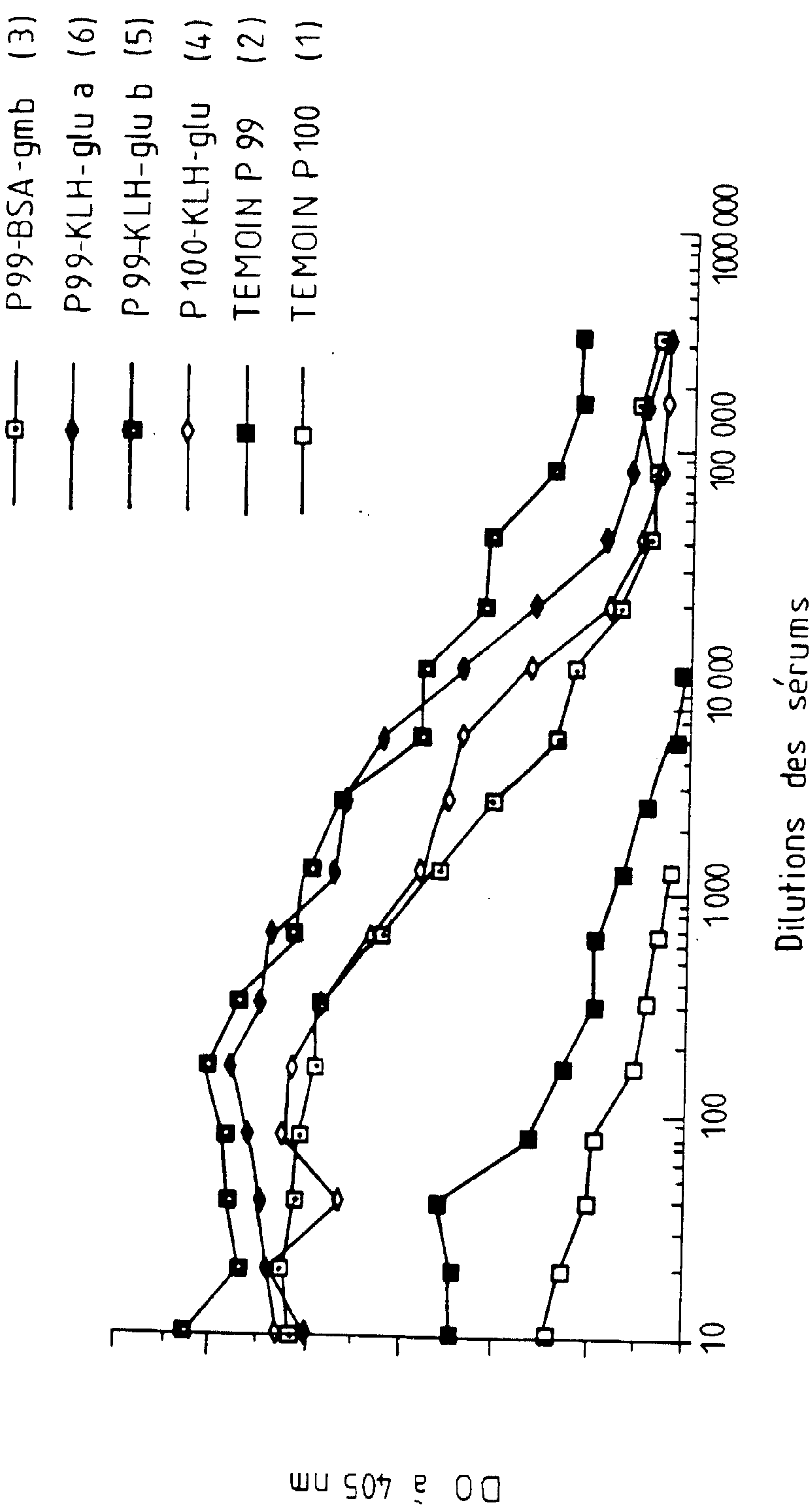
P99-BSA-gmb (3)
P99-KLH-glu a (6)
P 99-KLH-glu b (5)
P 100-KLH-glu (4)
TEMOIN P 99 (2)
TEMOIN P 100 (1)
DO à 405 nm
10
100
1000
10 000
100 000
1000 000
Dilutions des sérums

FIG. 6

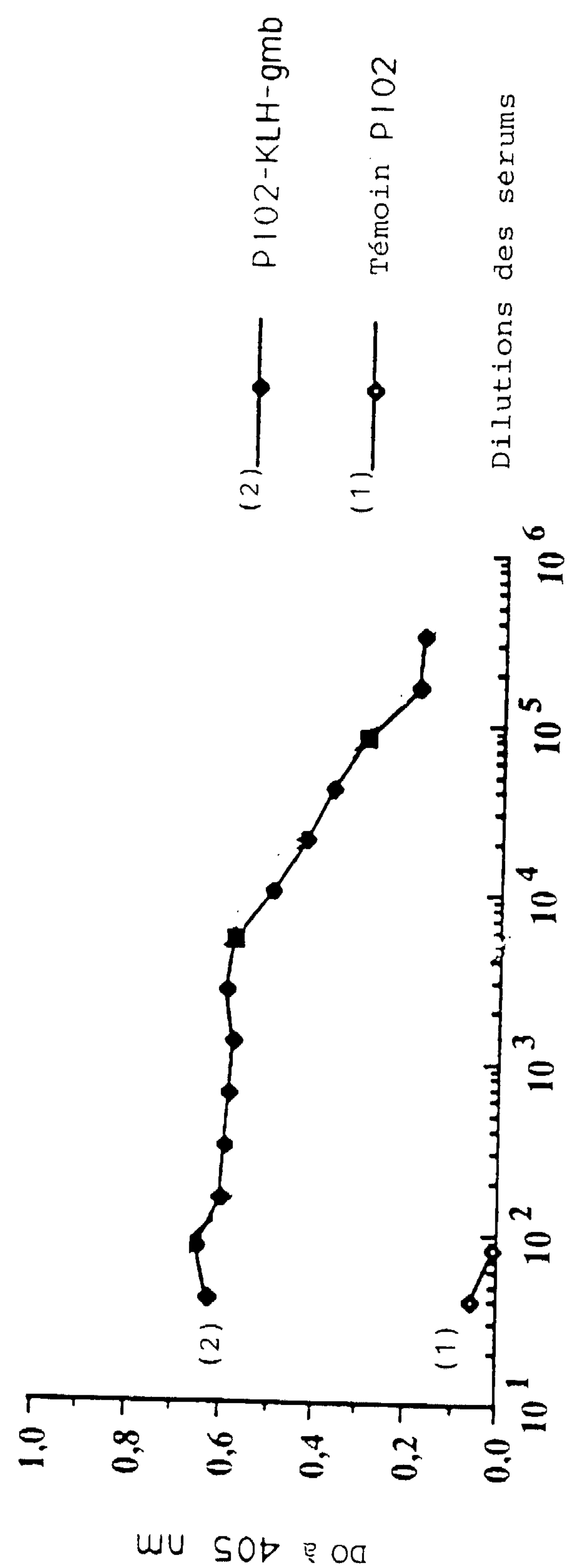
(2) P102-KLH-gmb
(1) Témoin P102
Dilutions des sérums
DO à 405 nm
1,0
0,8
0,6
0,4
0,2
0,0
$10^1$
$10^2$
$10^3$
$10^4$
$10^5$
$10^6$
(2)
(1)

FIGURE 7